# EUROPEAN PATENT APPLICATION

(11) **EP 3 067 058 A1**
(43) Date of publication of application: **14.09.2016**
(21) Application number: 16159001.3
(22) Date of filing: 07.03.2016
(51) Int. Cl.: A61K 35/747, C12N 15/52

(54) **BIOLOGICAL COMPOSITION BASED ON ENGINEERED LACTOBACILLUS PARACASEI SUBSP. PARACASEI F19 FOR THE BIOSYNTHESIS OF CANNABINOIDS**

(30) Priority: 13.03.2015 IT RM20150107
(71) Applicant: Farmagens Health Care Srl, 00191 Rome (IT)
(72) Inventor: Sannetti, Augusto, I-00168 Rome (IT); Cuomo, Rosario, I-81100 Caserta (IT); Sarnelli, Giovanni, I-80078 Pozzuoli (NA) (IT); Esposito, Giuseppe, I-81100 Caserta (IT); Lu, Jie, Mountain View, CA 94040 (US)
(74) Representative: Sarpi, Maurizio

(57) **Abstract**

The invention provides a method for producing therapeutic cannabinoids characterized by administering to an host the probiotic *Lactobacillus Paracasei* subsp. Paracasei F19, genetically modified to produce and secrete cannabinoids from *Cannabis sativa,* in association with caproic acid, able to establish the enzymatic reactions of the biosynthetic pathway leading to the production of cannabinoids directly *in situ* in said host.

SEQUENCE LISTING

<110> FARMAGENS HEALTHCARE SRL

<120> BIOLOGICAL COMPOSITION BASED ON ENGINEERED LACTOBACILLUS PARACASEI
SUBSP. PARACASEI F19 FOR THE BIOSYNTHESIS OF CANNABINOIDS

<130> 9342

<150> PCT/ITRM2015A000107
<151> 2015-03-13

<160> 5

<170> BiSSAP 1.3

<210> 1
<211> 385
<212> PRT
<213> Cannabis sativa

<400> 1
Met Asn His Leu Arg Ala Glu Gly Pro Ala Ser Val Leu Ala Ile Gly
1               5                   10                  15
Thr Ala Asn Pro Glu Asn Ile Leu Leu Gln Asp Glu Phe Pro Asp Tyr
            20                  25                  30
Tyr Phe Arg Val Thr Lys Ser Glu His Met Thr Gln Leu Lys Glu Lys
        35                  40                  45
Phe Arg Lys Ile Cys Asp Lys Ser Met Ile Arg Lys Arg Asn Cys Phe
    50                  55                  60
Leu Asn Glu Glu His Leu Lys Gln Asn Pro Arg Leu Val Glu His Glu
65                  70                  75                  80
Met Gln Thr Leu Asp Ala Arg Gln Asp Met Leu Val Val Glu Val Pro
                85                  90                  95
Lys Leu Gly Lys Asp Ala Cys Ala Lys Ala Ile Lys Glu Trp Gly Gln
            100                 105                 110
Pro Lys Ser Lys Ile Thr His Leu Ile Phe Thr Ser Ala Ser Thr Thr
        115                 120                 125
Asp Met Pro Gly Ala Asp Tyr His Cys Ala Lys Leu Leu Gly Leu Ser
    130                 135                 140
Pro Ser Val Lys Arg Val Met Met Tyr Gln Leu Gly Cys Tyr Gly Gly
145                 150                 155                 160
Gly Thr Val Leu Arg Ile Ala Lys Asp Ile Ala Glu Asn Asn Lys Gly
                165                 170                 175
Ala Arg Val Leu Ala Val Cys Cys Asp Ile Met Ala Cys Leu Phe Arg
            180                 185                 190
Gly Pro Ser Glu Ser Asp Leu Glu Leu Leu Val Gly Gln Ala Ile Phe
        195                 200                 205
Gly Asp Gly Ala Ala Ala Val Ile Val Gly Ala Glu Pro Asp Glu Ser
    210                 215                 220
Val Gly Glu Arg Pro Ile Phe Glu Leu Val Ser Thr Gly Gln Thr Ile
225                 230                 235                 240
Leu Pro Asn Ser Glu Gly Thr Ile Gly Gly His Ile Arg Glu Ala Gly
                245                 250                 255
Leu Ile Phe Asp Leu His Lys Asp Val Pro Met Leu Ile Ser Asn Asn
            260                 265                 270
Ile Glu Lys Cys Leu Ile Glu Ala Phe Thr Pro Ile Gly Ile Ser Asp
        275                 280                 285
Trp Asn Ser Ile Phe Trp Ile Thr His Pro Gly Gly Lys Ala Ile Leu
    290                 295                 300
Asp Lys Val Glu Glu Lys Leu His Leu Lys Ser Asp Lys Phe Val Asp
305                 310                 315                 320
Ser Arg His Val Leu Ser Glu His Gly Asn Met Ser Ser Ser Thr Val

                325                 330                 335
Leu Phe Val Met Asp Glu Leu Arg Lys Arg Ser Leu Glu Glu Gly Lys
            340                 345                 350
Ser Thr Thr Gly Asp Gly Phe Glu Trp Gly Val Leu Phe Gly Phe Gly
        355                 360                 365
Pro Gly Leu Thr Val Glu Arg Val Val Val Arg Ser Val Pro Ile Lys
    370                 375                 380
Tyr
385

<210> 2
<211> 101
<212> PRT
<213> Cannabis sativa

<400> 2
Met Ala Val Lys His Leu Ile Val Leu Lys Phe Lys Asp Glu Ile Thr
1               5                   10                  15
Glu Ala Gln Lys Glu Glu Phe Phe Lys Thr Tyr Val Asn Leu Val Asn
            20                  25                  30
Ile Ile Pro Ala Met Lys Asp Val Tyr Trp Gly Lys Asp Val Thr Gln
        35                  40                  45
Lys Asn Lys Glu Glu Gly Tyr Thr His Ile Val Glu Val Thr Phe Glu
    50                  55                  60
Ser Val Glu Thr Ile Gln Asp Tyr Ile Ile His Pro Ala His Val Gly
65                  70                  75                  80
Phe Gly Asp Val Tyr Arg Ser Phe Trp Glu Lys Leu Leu Ile Phe Asp
                85                  90                  95
Tyr Thr Pro Arg Lys
            100

<210> 3
<211> 395
<212> PRT
<213> Cannabis sativa

<400> 3
Met Gly Leu Ser Ser Val Cys Thr Phe Ser Phe Gln Thr Asn Tyr His
1               5                   10                  15
Thr Leu Leu Asn Pro His Asn Asn Asn Pro Lys Thr Ser Leu Leu Cys
            20                  25                  30
Tyr Arg His Pro Lys Thr Pro Ile Lys Tyr Ser Tyr Asn Asn Phe Pro
        35                  40                  45
Ser Lys His Cys Ser Thr Lys Ser Phe His Leu Gln Asn Lys Cys Ser
    50                  55                  60
Glu Ser Leu Ser Ile Ala Lys Asn Ser Ile Arg Ala Ala Thr Thr Asn
65                  70                  75                  80
Gln Thr Glu Pro Pro Glu Ser Asp Asn His Ser Val Ala Thr Lys Ile
                85                  90                  95
Leu Asn Phe Gly Lys Ala Cys Trp Lys Leu Gln Arg Pro Tyr Thr Ile
            100                 105                 110
Ile Ala Phe Thr Ser Cys Ala Cys Gly Leu Phe Gly Lys Glu Leu Leu
        115                 120                 125
His Asn Thr Asn Leu Ile Ser Trp Ser Leu Met Phe Lys Ala Phe Phe
    130                 135                 140
Phe Leu Val Ala Ile Leu Cys Ile Ala Ser Phe Thr Thr Thr Ile Asn
145                 150                 155                 160
Gln Ile Tyr Asp Leu His Ile Asp Arg Ile Asn Lys Pro Asp Leu Pro
                165                 170                 175
Leu Ala Ser Gly Glu Ile Ser Val Asn Thr Ala Trp Ile Met Ser Ile
            180                 185                 190
Ile Val Ala Leu Phe Gly Leu Ile Ile Thr Ile Lys Met Lys Gly Gly
        195                 200                 205

Pro Leu Tyr Ile Phe Gly Tyr Cys Phe Gly Ile Phe Gly Gly Ile Val
    210                 215                 220
Tyr Ser Val Pro Pro Phe Arg Trp Lys Gln Asn Pro Ser Thr Ala Phe
225                 230                 235                 240
Leu Leu Asn Phe Leu Ala His Ile Ile Thr Asn Phe Thr Phe Tyr Tyr
                245                 250                 255
Ala Ser Arg Ala Ala Leu Gly Leu Pro Phe Glu Leu Arg Pro Ser Phe
            260                 265                 270
Thr Phe Leu Leu Ala Phe Met Lys Ser Met Gly Ser Ala Leu Ala Leu
        275                 280                 285
Ile Lys Asp Ala Ser Asp Val Glu Gly Asp Thr Lys Phe Gly Ile Ser
    290                 295                 300
Thr Leu Ala Ser Lys Tyr Gly Ser Arg Asn Leu Thr Leu Phe Cys Ser
305                 310                 315                 320
Gly Ile Val Leu Leu Ser Tyr Val Ala Ala Ile Leu Ala Gly Ile Ile
                325                 330                 335
Trp Pro Gln Ala Phe Asn Ser Asn Val Met Leu Leu Ser His Ala Ile
            340                 345                 350
Leu Ala Phe Trp Leu Ile Leu Gln Thr Arg Asp Phe Ala Leu Thr Asn
        355                 360                 365
Tyr Asp Pro Glu Ala Gly Arg Arg Phe Tyr Glu Phe Met Trp Lys Leu
    370                 375                 380
Tyr Tyr Ala Glu Tyr Leu Val Tyr Val Phe Ile
385                 390                 395

<210> 4
<211> 544
<212> PRT
<213> Cannabis sativa

<400> 4
Met Lys Cys Ser Thr Phe Ser Phe Trp Phe Val Cys Lys Ile Ile Phe
1               5                   10                  15
Phe Phe Phe Ser Phe Asn Ile Gln Thr Ser Ile Ala Asn Pro Arg Glu
            20                  25                  30
Asn Phe Leu Lys Cys Phe Ser Gln Tyr Ile Pro Asn Asn Ala Thr Asn
        35                  40                  45
Leu Lys Leu Val Tyr Thr Gln Asn Asn Pro Leu Tyr Met Ser Val Leu
    50                  55                  60
Asn Ser Thr Ile His Asn Leu Arg Phe Thr Ser Asp Thr Thr Pro Lys
65                  70                  75                  80
Pro Leu Val Ile Val Thr Pro Ser His Val Ser His Ile Gln Gly Thr
                85                  90                  95
Ile Leu Cys Ser Lys Lys Val Gly Leu Gln Ile Arg Thr Arg Ser Gly
            100                 105                 110
Gly His Asp Ser Glu Gly Met Ser Tyr Ile Ser Gln Val Pro Phe Val
        115                 120                 125
Ile Val Asp Leu Arg Asn Met Arg Ser Ile Lys Ile Asp Val His Ser
    130                 135                 140
Gln Thr Ala Trp Val Glu Ala Gly Ala Thr Leu Gly Glu Val Tyr Tyr
145                 150                 155                 160
Trp Val Asn Glu Lys Asn Glu Asn Leu Ser Leu Ala Ala Gly Tyr Cys
                165                 170                 175
Pro Thr Val Cys Ala Gly Gly His Phe Gly Gly Gly Gly Tyr Gly Pro
            180                 185                 190
Leu Met Arg Asn Tyr Gly Leu Ala Ala Asp Asn Ile Ile Asp Ala His
        195                 200                 205
Leu Val Asn Val His Gly Lys Val Leu Asp Arg Lys Ser Met Gly Glu
    210                 215                 220
Asp Leu Phe Trp Ala Leu Arg Gly Gly Gly Ala Glu Ser Phe Gly Ile
225                 230                 235                 240
Ile Val Ala Trp Lys Ile Arg Leu Val Ala Val Pro Lys Ser Thr Met
                245                 250                 255

Phe Ser Val Lys Lys Ile Met Glu Ile His Glu Leu Val Lys Leu Val
                260                 265                 270
Asn Lys Trp Gln Asn Ile Ala Tyr Lys Tyr Asp Lys Asp Leu Leu Leu
        275                 280                 285
Met Thr His Phe Ile Thr Arg Asn Ile Thr Asp Asn Gln Gly Lys Asn
    290                 295                 300
Lys Thr Ala Ile His Thr Tyr Phe Ser Ser Val Phe Leu Gly Gly Val
305                 310                 315                 320
Asp Ser Leu Val Asp Leu Met Asn Lys Ser Phe Pro Glu Leu Gly Ile
                325                 330                 335
Lys Lys Thr Asp Cys Arg Gln Leu Ser Trp Ile Asp Thr Ile Ile Phe
                340                 345                 350
Tyr Ser Gly Val Val Asn Tyr Asp Thr Asp Asn Phe Asn Lys Glu Ile
        355                 360                 365
Leu Leu Asp Arg Ser Ala Gly Gln Asn Gly Ala Phe Lys Ile Lys Leu
    370                 375                 380
Asp Tyr Val Lys Lys Pro Ile Pro Glu Ser Val Phe Val Gln Ile Leu
385                 390                 395                 400
Glu Lys Leu Tyr Glu Glu Asp Ile Gly Ala Gly Met Tyr Ala Leu Tyr
                405                 410                 415
Pro Tyr Gly Gly Ile Met Asp Glu Ile Ser Glu Ser Ala Ile Pro Phe
                420                 425                 430
Pro His Arg Ala Gly Ile Leu Tyr Glu Leu Trp Tyr Ile Cys Ser Trp
        435                 440                 445
Glu Lys Gln Glu Asp Asn Glu Lys His Leu Asn Trp Ile Arg Asn Ile
    450                 455                 460
Tyr Asn Phe Met Thr Pro Tyr Val Ser Lys Asn Pro Arg Leu Ala Tyr
465                 470                 475                 480
Leu Asn Tyr Arg Asp Leu Asp Ile Gly Ile Asn Asp Pro Lys Asn Pro
                485                 490                 495
Asn Asn Tyr Thr Gln Ala Arg Ile Trp Gly Glu Lys Tyr Phe Gly Lys
                500                 505                 510
Asn Phe Asp Arg Leu Val Lys Val Lys Thr Leu Val Asp Pro Asn Asn
        515                 520                 525
Phe Phe Arg Asn Glu Gln Ser Ile Pro Pro Leu Pro Arg His Arg His
    530                 535                 540

<210> 5
<211> 544
<212> PRT
<213> Cannabis sativa

<400> 5
Met Lys Cys Ser Thr Phe Ser Phe Trp Phe Val Cys Lys Ile Ile Phe
1               5                   10                  15
Phe Phe Phe Ser Phe Asn Ile Gln Thr Ser Ile Ala Asn Pro Arg Glu
                20                  25                  30
Asn Phe Leu Lys Cys Phe Ser Gln Tyr Ile Pro Asn Asn Ala Thr Asn
        35                  40                  45
Leu Lys Leu Val Tyr Thr Gln Asn Asn Pro Leu Tyr Met Ser Val Leu
    50                  55                  60
Asn Ser Thr Ile His Asn Leu Arg Phe Thr Ser Asp Thr Thr Pro Lys
65                  70                  75                  80
Pro Leu Val Ile Val Thr Pro Ser His Val Ser His Ile Gln Gly Thr
                85                  90                  95
Ile Leu Cys Ser Lys Lys Val Gly Leu Gln Ile Arg Thr Arg Ser Gly
                100                 105                 110
Gly His Asp Ser Glu Gly Met Ser Tyr Ile Ser Gln Val Pro Phe Val
        115                 120                 125
Ile Val Asp Leu Arg Asn Met Arg Ser Ile Lys Ile Asp Val His Ser
    130                 135                 140

Gln Thr Ala Trp Val Glu Ala Gly Ala Thr Leu Gly Glu Val Tyr Tyr
145                 150                 155                 160
Trp Val Asn Glu Lys Asn Glu Asn Leu Ser Leu Ala Ala Gly Tyr Cys
                165                 170                 175
Pro Thr Val Cys Ala Gly Gly His Phe Gly Gly Gly Gly Tyr Gly Pro
            180                 185                 190
Leu Met Arg Asn Tyr Gly Leu Ala Ala Asp Asn Ile Ile Asp Ala His
        195                 200                 205
Leu Val Asn Val His Gly Lys Val Leu Asp Arg Lys Ser Met Gly Glu
    210                 215                 220
Asp Leu Phe Trp Ala Leu Arg Gly Gly Gly Ala Glu Ser Phe Gly Ile
225                 230                 235                 240
Ile Val Ala Trp Lys Ile Arg Leu Val Ala Val Pro Lys Ser Thr Met
                245                 250                 255
Phe Ser Val Lys Lys Ile Met Glu Ile His Glu Leu Val Lys Leu Val
            260                 265                 270
Asn Lys Trp Gln Asn Ile Ala Tyr Lys Tyr Asp Lys Asp Leu Leu Leu
        275                 280                 285
Met Thr His Phe Ile Thr Arg Asn Ile Thr Asp Asn Gln Gly Lys Asn
    290                 295                 300
Lys Thr Ala Ile His Thr Tyr Phe Ser Ser Val Phe Leu Gly Gly Val
305                 310                 315                 320
Asp Ser Leu Val Asp Leu Met Asn Lys Ser Phe Pro Glu Leu Gly Ile
                325                 330                 335
Lys Lys Thr Asp Cys Arg Gln Leu Ser Trp Ile Asp Thr Ile Ile Phe
            340                 345                 350
Tyr Ser Gly Val Val Asn Tyr Asp Thr Asp Asn Phe Asn Lys Glu Ile
        355                 360                 365
Leu Leu Asp Arg Ser Ala Gly Gln Asn Gly Ala Phe Lys Ile Lys Leu
    370                 375                 380
Asp Tyr Val Lys Lys Pro Ile Pro Glu Ser Val Phe Val Gln Ile Leu
385                 390                 395                 400
Glu Lys Leu Tyr Glu Glu Asp Ile Gly Ala Gly Met Tyr Ala Leu Tyr
                405                 410                 415
Pro Tyr Gly Gly Ile Met Asp Glu Ile Ser Glu Ser Ala Ile Pro Phe
            420                 425                 430
Pro His Arg Ala Gly Ile Leu Tyr Glu Leu Trp Tyr Ile Cys Ser Trp
        435                 440                 445
Glu Lys Gln Glu Asp Asn Glu Lys His Leu Asn Trp Ile Arg Asn Ile
    450                 455                 460
Tyr Asn Phe Met Thr Pro Tyr Val Ser Lys Asn Pro Arg Leu Ala Tyr
465                 470                 475                 480
Leu Asn Tyr Arg Asp Leu Asp Ile Gly Ile Asn Asp Pro Lys Asn Pro
                485                 490                 495
Asn Asn Tyr Thr Gln Ala Arg Ile Trp Gly Glu Lys Tyr Phe Gly Lys
            500                 505                 510
Asn Phe Asp Arg Leu Val Lys Val Lys Thr Leu Val Asp Pro Asn Asn
        515                 520                 525
Phe Phe Arg Asn Glu Gln Ser Ile Pro Pro Leu Pro Arg His Arg His
    530                 535                 540

## Description

### Field of the invention

The present invention relates to the field of biological compositions that use genetically engineered probiotics, or their metabolites, to enhance the benefits arising from the use of the probiotic, orienting the biosynthesis of therapeutic substances based on the real needs of individuals.

### Background of the invention

In Europe, the use of preparation of *Cannabis sativa* was introduced in nineteenth century following the conquest of the territories of the Ottoman Empire by Napoleon's troops. Afterwards, cannabis was the object of clinical attention. William O'Shaughnessy in 1842 introduced the use of cannabis to treat various diseases (pain, muscle spasms, convulsions, rheumatism, epilepsy), thus becoming part of Western medicine. Already at that time the cannabis preparations had applications in the field of gastrointestinal disorders: abdominal pain, diarrhea and gastroenteritis and to relieve the symptoms of Crohn's disease and diabetic gastroparesis. However, over the time due to the unpredictability of the action of the extracts, the extreme variability of their power, the inability to optimize the dosage and the presence of side effects due to the action on the central nervous system, the therapeutic use of cannabis preparations for gastrointestinal disorders has been discouraged and abandoned. With the advent of synthetic drugs (anesthetics, aspirin, barbiturates and benzodiazepines, phenothiazines) cannabis preparations went quickly into disuse, and its reputation was tied to the illegal use. In the second half of the twentieth century by developing the isolation procedure for cannabinoids the interest of the scientific community towards the *Cannabis sativa* extracts has renewed. More than 70 cannabinoids have been identified and among them the most abundant is the Δ9-tetrahydrocannabinol (Δ⁹-THC), considered the main but not the only one responsible for the pharmacological effects. Other constituents are cannabinol, cannabidiol, cannabigerol, cannabichromene and related acids.

Cannabidiol (CBD) is one of at least 85 active cannabinoids identified in *Cannabis sativa* (Borgelt et al., 2013. Pharmacotherapy (Review) 33: 195-209). It is a major phytocannabinoid, accounting for up to 40% of the plant's extract (Campos et al., 2012. Philos. TransR. Soc. Lond., B, Biol. Sci. (Review) 367: 3364-78).

The pharmacological properties of cannabidiol have been extensively investigated; CBD exerts antinflammatory, antioxidant and anti-apoptotic effects (Iuvone et al., 2009. CNS Neurosci Ther. 15:65-75). Importantly, CBD has been demonstrated to display potent anti-inflammatory and immunomodulatory properties which, together with a lack of psychotropic activity and low toxicity (Mechoulam and Hanus, 2002. Chem Phys Lipids. 121:35-43) make it a very promising therapeutic candidate for a variety of inflammatory and pain associated disorders, including inflammatory bowel disease (IBD). Many protective functions of CBD have been related to the impressive antioxidant function displayed by CBD during inflammation; such antioxidant activity has been reported to markedly inhibit colon injury, inducible iNOS expression, interleukin-1β, interleukin-10. Further effects of CBD on immune cells include interferon (IFN)-γ release inhibition by mononuclear cells (Formukong et al., 1988. Inflammation. 12:361-371; Watzl et al., 1991. Int J Immunopharmacol. 13:1091-1097), and the suppression of chemokine production by human B cells (Srivastava et al., 1998. Immunopharmacology. 40:179-185). These observations account for the great efficacy in decreasing the severity of experimental colitis in rodents (Borrelli et al., 2009. J Mol Med (Berl). 87: 1111-21; Shicho and Storr, 2012. Pharmacology. 89: 149-55.). CBD-mediated activation of PPARγ (O'Sullivan et al., 2009. Eur J Pharmacol. 612: 61-8) may represent a key mechanism that explains the potent antinflammatory action of this compound in IBD. Peroxisome proliferator-activated receptor-gamma has an inestimable value as target for novel therapeutics against IBD (Lewis et al., 2011. PLoS One 6(8):e24031).

Directly related to IBD, colon cancer is a major cause of morbidity and mortality in Western countries. It has been demonstrated that CBD is able to exert a profound anticancer activity in an animal model of colon cancer based on azoxymethane (AOM) administration in mice (Aviello et al., 2012. J Mol Med (Berl); 90:925-34). AOM treatment was associated with Aberrant Crypt Foci (ACF), polyps, and tumour formation, as well as with the upregulation of phospho-Akt, iNOS and COX-2 and the downregulation of caspase-3.

*In vitro* studies supported the beneficial effect of CBD. In colorectal carcinoma cell lines, CBD protected DNA from oxidative damage. These results suggest that CBD might be worthy of clinical consideration in colon cancer prevention.

Furthermore, cannabidiol (CBD) is known to exhibit neuroprotective, anti-inflammatory and anti-oxidant properties in models of neuro-toxicity and neurodegeneration. Promising results have been achieved with CBD in the control of toxicity induced by β-amyloid peptide, which is responsible for the neurodegeneration in Alzheimer's patients (Iuvone et al. 2009).

The beneficial effects of CBD in the treatment of neurodegenerative diseases in an in vivo model of Alzheimer's disease have also been demonstrated (Esposito et al. 2007. Mol Immunol. Epub, 2007 Sep 11).

Although CBD is a well drug tolerated in humans, lacking any toxicity profile in clinical trials where it has been tested, CBD has not been prompted in clinical practice since one of the most limiting aspects for its entry in therapy is represented by costs of production, importation, extraction and purification by *Cannabis sativa.*

Tetrahydrocannabinol (THC), or more precisely its main isomer (-)-trans-Δ9-tetrahydrocannabinol ((6aR,10aR)-delta-9-tetrahydrocannabinol), is the principal psychoactive constituent (or cannabinoid) of cannabis. THC has mild to moderate analgesic effects. Other effects include relaxation, alteration of visual, auditory, and olfactory senses, fatigue, and appetite stimulation. THC has marked antiemetic properties. It may acutely reduce aggression and increase aggression during withdrawal. Evidence suggests that THC helps alleviating symptoms suffered both by AIDS patients, and by cancer patients undergoing chemotherapy. THC is used by a number of multiple sclerosis patients, who use it to alleviate neuropathic pain and spasticity.

The United Kingdom patent, GB2432312, has demonstrated that the administration of cannabinoids, as extracts from cannabis plants, are more efficacious than the purified compounds, in the prevention of neural degeneration. In particular cannabinoid-rich extracts comprising as a predominant cannabinoid either tetrahydrocannabinol (THC) or cannabidiol (CBD) were particularly efficacious in the prevention of neurodegeneration. The applicants additionally found that a combination of the two cannabinoid extracts were particularly effective neuroprotectants.

Beside cannabidiol and tetrahydrocannabinol there are at least 85 different cannabinoids isolated from cannabis, exhibiting varied effects, but their possible therapeutic use needs to be confirmed by further medical studies.

The UK patent application GB2479153 demonstrates the anti-epileptic effects of the phytocannabinoid cannabidivarin (CBDV).

The patent application GB2377633 discusses formulations which comprise THC and CBD and may also comprise CBDV.

Recently, has been granted the US patent application (US 8,632,825) covering the use of a combination of cannabinoids, particularly tetrahydrocannabinol (THC) and cannabidiol (CBD), for the production of a medicament for the use in the treatment of cancer. In particular, the cancer to be treated is a brain tumor, more particularly a glioma, more particularly still a glioblastoma multiforme (GBM). The method for treating glioblastoma multiforme in a human in need thereof consisting essentially of administering to said human therapeutically effective amounts of cannabidiol (CBD) and tetrahydrocannabinol (THC) in combination as pure cannabinoids wherein the cannabinoids are in a ratio of from 1:1 to 1:20 (THC:CBD).

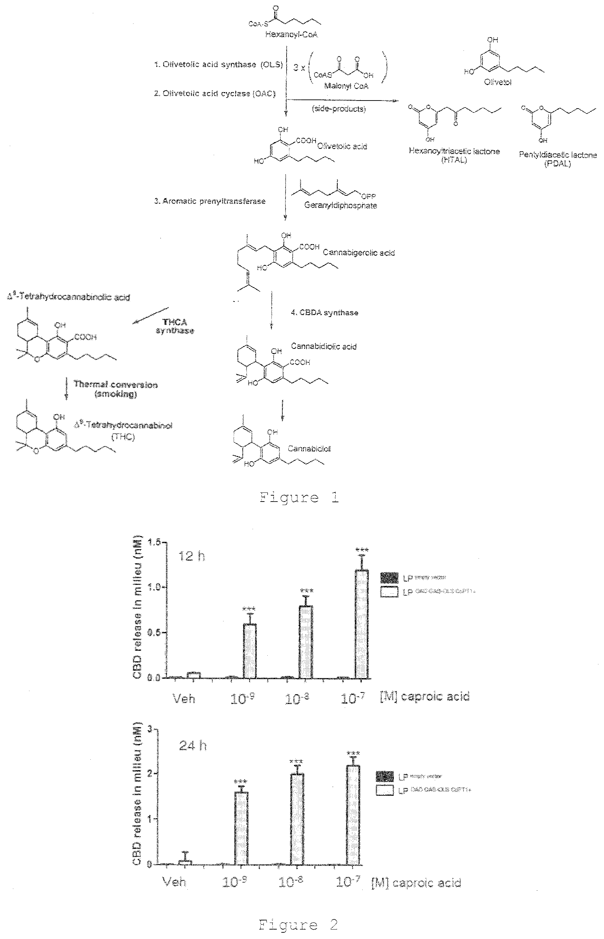

Cannabinoids have their biosynthetic origin in both polyketide (phenolic) and terpenoid methabolism and are termed terpenophenolics or prenylated polyketides (Page and Nagel, 2006). Cannabinoids biosynthesis occurs primarily in glandular trichomes that cover female flowers at a high density. Cannabinoids are formed by a three step biosynthetic process: polyketide formation, aromatic prenylation and cyclization (Fig. 1). The first enzyme in the cannabinoid biosynthetic pathway is a type III polyketide synthase (PKS) that catalyzes the condensation of hexanoyl-CoA with three molecules of malonyl-CoA to yield olivetolic acid (OA). Two sequential enzymes are involved in the synthesis of olivetolic acid, respectively, OA synthase and OA cyclase synthase. The final product of these reactions is olivetolic acid, as stable intermediate of the biosynthetic pathway. Subsequently, this compound is thus geranylated to form cannabigerolic acid (CBGA) (Fellermeier et al., 1998) through the intervention of a specific Aromatic prenyltransferase (GPP, olivetolate geranyltransferase). Cannabigerolic acid could be converted by oxidocyclase enzymes to the major cannabinoids, Δ9-tetrahydrocannabinolic acid (THCA) and cannabidiolic acid (CBDA) (Sirikantaramas et al., 2004; Taura et al., 2007) by specific intervention of THCA synthase or CBDA synthase. By spontaneous decarboxylation respectively products will be converted in, THC and cannabidiol (CBD), respectively.

The pharmacological effects of cannabis extract vary depending on the dose, route of administration, the vulnerability to the psychoactive effects and conditions of use. The most common and apparently more effective method of administration is smoking: about 20% of Δ⁹-THC content in a cannabis cigarette is absorbed. The effects appear quickly and last for about two hours. The parenteral administration, practically unused, has even shorter effect. Its oral administration is less effective and the bioavailability is very variable with respect to smoking (Mechoulam, 1996). Hence, the cannabis effects vary greatly in relation to the content of active ingredients in the preparation, methods of administration, the speed with which it is metabolized, the duration and intensity of use/abuse.

In order to maintain the beneficial therapeutic effects on target organ, and in particular on gut inflammation of cannabinoids overcoming the drawbacks related the great variability of the action of the active principles, the difficulties in optimizing the dosage and the presence of systemic side effects, and to assure the major bioavailability on site, the Applicant proposes the use of a probiotic microorganism genetically modified to overexpress directly in the gut four enzymes involved in the cannabinoid synthesis pathway. Under an increased external supply of caproic acid the multiple engineered probiotic will be able to produce a significant amount of therapeutic cannabinoids *in situ*.

With respect to other prior art solutions the present invention shows evident innovative aspects and provide advantages not derivable from early teachings. Namely, the international patent application WO 2011/127589 disclosing the overexpression in yeast or *E. coli* of olivetol synthase, or maybe more correctly olivetolic acid cyclase from *Cannabis sativa,* according to Gagne S.J. et al. 2012. Proc. Natl. Acad. Sci. U.S.A. 109:12811-12816, includes as part of the description many variations of the gene sequence and of the corresponding codon sequence, in particular the codon-optimized sequence using codons known to provide higher expression in *E. coli*. According to the author's statements the expression or over-expression of the nucleic acid molecules of the invention will result in the expression or over-expression of the enzyme catalyzing the synthesis of aromatic polyketides (e.g. olivetolic acid) which may result in increased production of cannabinoid compounds such as cannabigerolic acid (CBGA), Δ9-tetrahydrocannabinolic acid (THCA), cannabidiolic acid (CBDA), cannabichromenic acid (CBCA), Δ9-tetrahydrocannabinol, cannabidiol, cannabichromene, etc. Similarly, depending on the substrate used, expression or over-expression of the nucleic acid molecules of the invention resulting in expression or over-expression of the enzyme that catalyzes the synthesis of aromatic polyketides may result in increased production of analogs of cannabinoid compounds, or analogs of precursors of such compounds. However, neither indications about how such results can be obtained are provided, nor hints about a possible modulation of the different cannabinoid compounds produced.

Furthermore, according to WO 2011/127589 the expression or over-expression of the nucleic acid molecules of the invention may be done in combination with the expression or over-expression of one or more other nucleic acids that encode one or more enzymes in a cannabinoid biosynthetic pathway. Some examples of other nucleic acids include: acyl CoA synthetase synthetase, a type III polyketide synthase, a polyketide cyclase, an aromatic prenyltransferase and a cannabinoid-forming oxidocylase. Specific examples of these enzymes include hexanoyl CoA synthetase, a type III polyketide synthase/olivetol synthase, a geranylpyrophosphate:olivetolate geranyltransferase, a Δ9-tetrahydrocannabinolic acid synthase, a cannabidiolic acid synthase or a cannabichromenic acid synthase. However, in the description the statement remains as such and does not appear to be sufficient to enable the skilled person to carry out the teaching itself.

### Summary of invention

In a first aspect of the present invention there is provided an isolated or purified nucleic acid molecule comprising the nucleotide sequence from *Cannabis sativa* coding for olivetolic acid cyclase (OAC), the nucleotide sequence coding for cannabidiolic acid synthase (CAS), the nucleotide sequence coding for olivetol synthase (OLS), and the nucleotide sequence coding for aromatic prenyltransferase (CsPT1) or the nucleotide sequence coding for tetrahydrocannabinolic (THC) acid synthase (THCAS).

In a second aspect of the invention there is provided the Lactobacillus Paracasei subsp paracasei F19 transformed with the nucleotide vector carrying the nucleic acid molecule comprising a nucleotide sequence coding for olivetolic acid cyclase (OAC), the nucleotide sequence coding for cannabidiolic acid synthase (CAS), the nucleotide sequence coding for olivetol synthase (OLS), and the nucleotide sequence encoding aromatic prenyltransferase (CsPT1) or the nucleotide sequence coding for THC acid synthase (THCAS) from *Cannabis sativa*.

In a third aspect of the present invention there is provided the composition comprising the *Lactobacillus Paracasei* subsp paracasei F19 transformed with a nucleotide vector comprising the nucleic acid coding for olivetolic acid cyclase (OAC), the nucleic acid coding for cannabidiolic acid synthase (CAS), the nucleic acid coding for olivetol synthase (OLS), and the nucleic acid coding for aromatic prenyltransferase (CsPT1) or THC acid synthase (THCAS) from *Cannabis sativa*

In a fourth aspect of the present invention there is provided a method for producing cannabinoids compound in an organism, cell or tissue comprising expressing or overexpressing the nucleic acid molecule of the present invention in the organism, cell or tissue. Hence, the invention provides a method for producing of therapeutic cannabinoids characterized by administering to an host the probiotic *Lactobacillus Paracasei* subsp. Paracasei F19, genetically modified to produce and secrete cannabinoids from *Cannabis sativa,* in association with caproic acid, able to establish the enzymatic reactions of the biosynthetic pathway leading to the production of cannabinoids directly *in situ* in said host.

Another object of the invention is the composition comprising Lactobacillus Paracasei subsp paracasei F19 transformed with a nucleotide vector comprising a nucleic acid coding for olivetolic acid cyclase (OAC), cannabidiolic acid synthase (CAS), olivetol synthase (OLS), and aromatic prenyltransferase (CsPT1) or THC acid synthase (THCAS) from *Cannabis sativa* for the use in the treatment of the gut inflammatory disorders.

A further object of the present invention is the composition comprising Lactobacillus Paracasei subsp. Paracasei F19, carrying four genes from *Cannabis sativa* selected from the group of genes coding for: olivetolic acid cyclase (OAC), cannabidiolic acid synthase (CAS), olivetol synthase (OLS), and aromatic prenyltransferase (CsPT1), or THC acid synthase (THCAS) associated with a dietary supplement of hexanoate that allows to obtain, high levels of intestinal cannabidiol for the use in the treatment of the gut inflammatory disorders, colonrectal cancer and neurodegeneration conditions, namely Alzheimer's disease.

Further characteristics of the invention will be described by the following detailed description.

### Brief description of drawings

In order to make the invention more clearly understandable, particularly preferred embodiments thereof will now be described in detail by way of example, with reference to the accompanying drawings.
Figure 1 depicts the scheme of the proposed pathway leading to the main cannabinoids in *Cannabis sativa* showing the central role of olivetol synthase (OLS), olivetolic acid cyclase (OAC), and aromatic prenyltransferase (CsPT1). The product obtained by these three sequential enzymatic reactions is the cannabigerolic acid. Depending on the following enzymatic action the cannabigerolic acid is subjected to, this can be transformed into cannabidiolic acid by cannabidiolic acid synthase (CAS), or into Δ⁹-tetrahydrocannabinolic acid by THCA synthase.

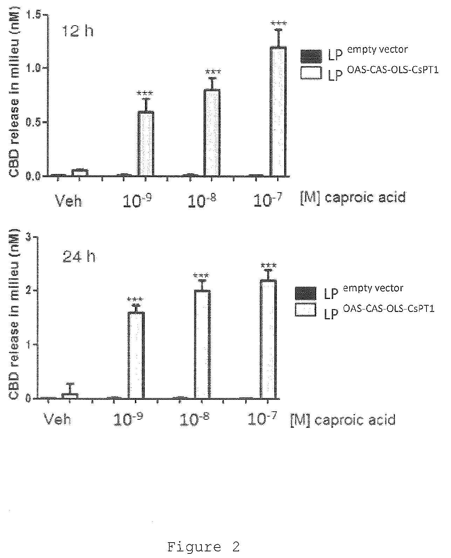

Figure 2 shows the release of CBD in the bacterial medium induced by LP-OAC-CAS-OLS-CsPT1⁺ in presence of caproic acid.

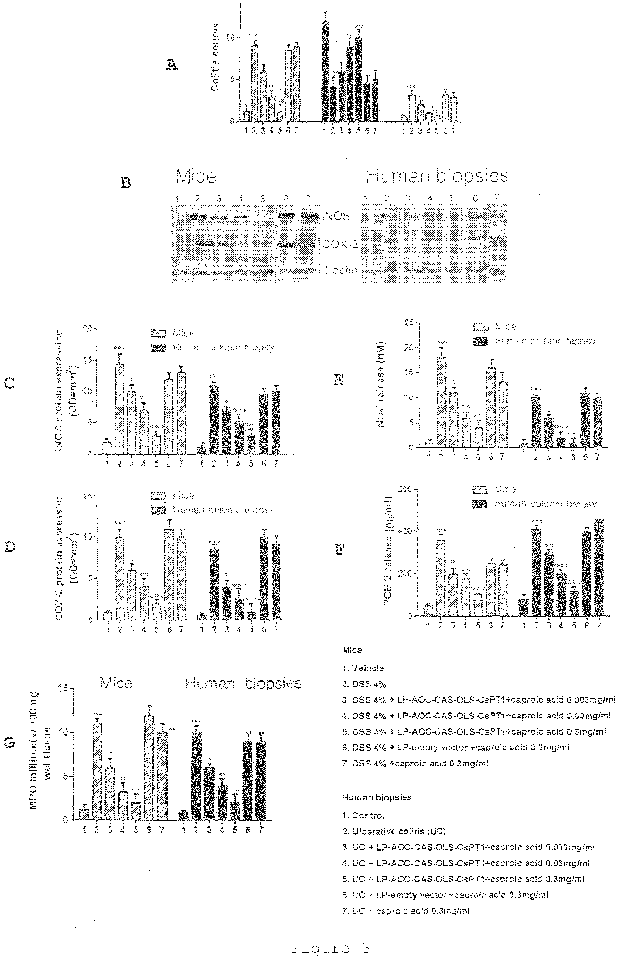

Figure 3 shows the effect of the administration of a composition comprising LP-OAC-CAS-OLS-CsPT1⁺ and caproic acid on a mice model of colitis (DSS induced). (3A) Disease Activity Index (DAI) (first series in the graph), colon shortening (second series in the graph) and splenomegaly (third series in the graph). (3B) Western blot analysis of iNOS and COX-2 protein carried out on mice model of colitis and human colonic biopsies subjected to the treatment. (3C) Graph showing the expression level of iNOS protein. (3D) Graph showing the expression level of COX-2 protein. (3E) Graph showing the release of NO₂ in colitis induced mice and in human colonic biopsies. (3F) Graph showing the release of PGE₂ in the same samples. (3G) Graph showing myeloperoxidase activity in the tested groups.

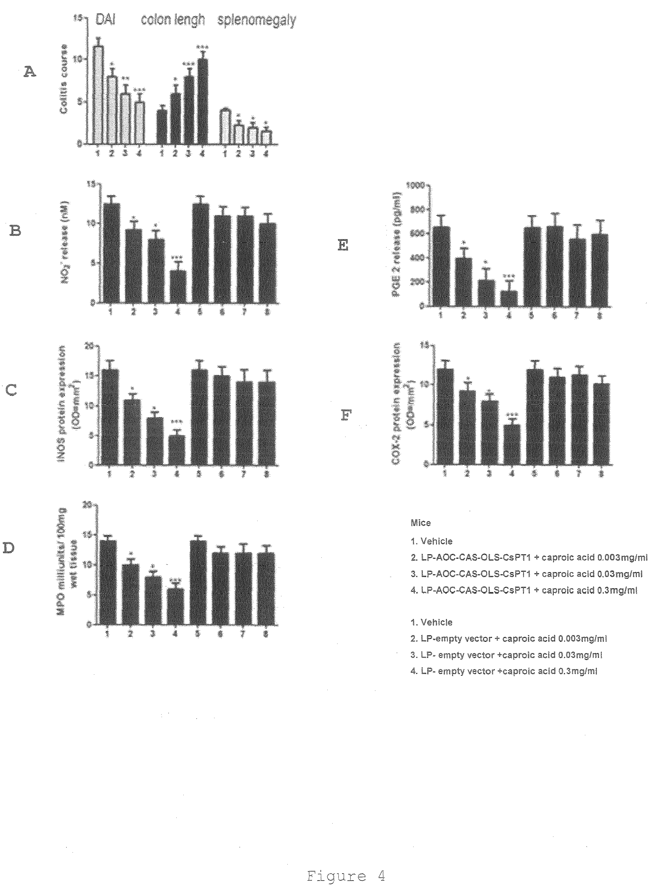

Figure 4 shows the effect provided by the administration of LP OAC-CAS-OLS-CsPT1+ in association with caproic acid in IL-10 KO chronic mice colitis model. (4A) Disease Activity Index (DAI), colon shortening and splenomegaly in experimental groups receiving the treatment according to the invention. (4B) Graph showing the release of NO₂ in chronic mice colitis. (4C) Graph showing the expression level of iNOS protein. (4D) Graph showing myeloperoxidase activity in the tested groups. (4E) Graph showing the release of PGE₂ in the same samples (4E). Graph showing the expression level of COX-2 protein.

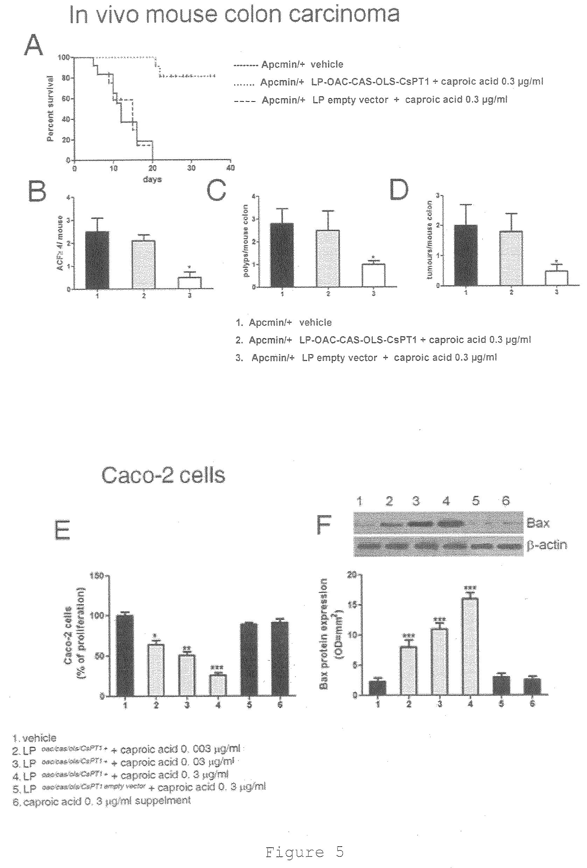

Figure 5 shows the anticancer effect determined by the administration of LP OAC-CAS-OLS-CsPT1+ and caproic acid both in a mouse colon carcinoma model and in human colon carcinoma cells. (5A) Graph showing the survival percentage of Apc^{min/+} mice receiving the treatment compared to tumour group and control mice (LP empty vector with caproic acid 0.3µg/ml supplement). (5B) Graph showing Aberrant Crypt Foci (ACF) in the experimental groups (5C). Graph showing polyps in mice colon. (5D) Graph showing tumors in the colon both versus tumour group and LP empty vector + caproic acid control. (5E) Graph showing the antiproliferative effect in the Caco-2 cell culture. (5F) Western blot and graph showing expression level of the pro-apoptotic protein Bax-2.

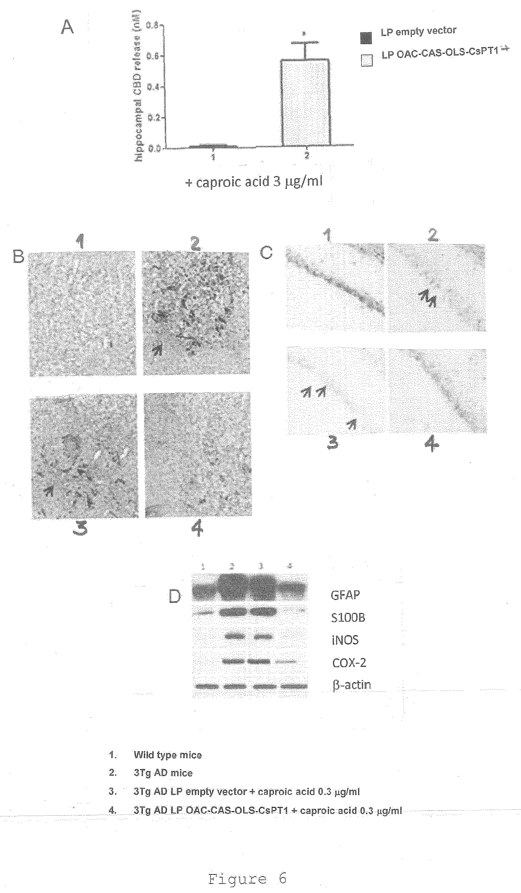

Figure 6 shows the neuroprotective effect of the co-administration of LP-OAC-CAS-OLS-CsPT1⁺ and caproic acid in a mouse model of Alzheimer disease. (6A) Graph showing the CBD release in the hippocampus of 3Tg AD mice. (6B) Histological analysis revealed the presence of plaques in brain sections of 3Tg AD mice. (6C) Pyramidal neurons of the hippocampal CA1 areas of 3Tg AD mice. (6D) Western blot analysis of reactive gliosis and pro-inflammatory proteins: GFAP, S100B, iNOS and COX-2 in the hippocampus.

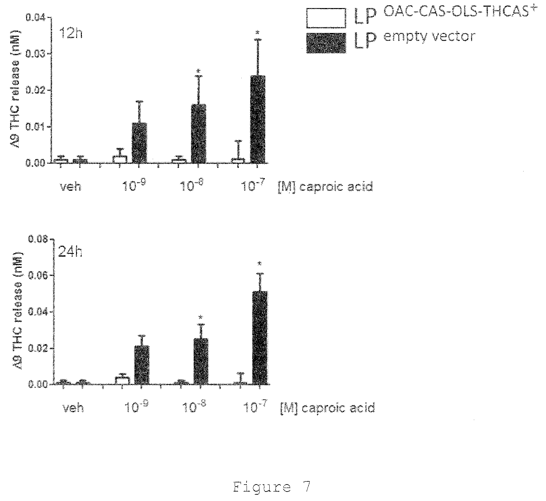

Figure 7 show the release of Δ9-THC in the bacterial medium induced by LP-OAC-CAS-OLS-CsPT1⁺ in presence of caproic acid.

### Detailed description of the invention

According to this invention the provision of a composition for cannabinoids production is achieved by the use of Lactobacillus Paracasei subsp. Paracasei F19, genetically engineered to carry more genes from *Cannabis sativa* selected from the group of genes coding for: olivetolic acid cyclase (OAC), cannabidiolic acid synthase (CAS), olivetol synthase (OLS), and aromatic prenyltransferase (CsPT1), or THC acid synthase (THCAS) associated with a dietary supplement of hexanoate that allows to obtain high levels of intestinal cannabidiol. Indeed, the use of this particular microorganism is not due to an alternative selection among several straightforward possibilities. The probiotic bacterium *Lactobacillus Paracasei* subsp paracasei F19 has demonstrated to be very stable in varying conditions of pH and not to be degraded by stomach acid and bile secretions, hence assuring that a larger amount of microorganism reach the target organ.

The beneficial properties of Lactobacillus Paracasei subsp. Paracasei F19 are known from US patent 6,599,504. Furthermore, this strain can act as a useful probiotic vector of natural therapeutic molecules, in fact, by transformation with specific DNA vectors the probiotic is able to produce directly in the human intestinal microbiota therapeutic molecules. Moreover, *Lactobacillus Paracasei* subsp paracasei F19 is able to ferment most carbohydrates, including lactose, galactose, tagatose, galacto-oligosaccharides, oligosaccharides, lactulose and all the carbohydrates present in the milk and its derivatives.

Some embodiment of the present invention relates to the probiotic *Lactobacillus Paracasei* subsp. Paracasei F19 transformed with a nucleic acid molecule, or DNA vector, construct or expression system, preferably a DNA bacterial expression vector, carrying four *Cannabis sativa* genes selected in the group of:
(i) gene coding for olivetol synthase (OLS) SEQ ID NO: 1,
(ii) gene coding for olivetolic acid cyclase (OAC) SEQ ID NO: 2,
(iii) gene coding for aromatic prenyltransferase (CsPT1) SEQ ID NO: 3,
(iv) gene coding for cannabidiolic acid synthase (CAS) SEQ ID NO: 4,
(v) gene coding for tetrahydrocannabinolic acid synthase (THCAS) SEQ ID NO: 5.

The olivetol synthase (OLS) from *Cannabis sativa* is a homodimeric protein that consists of a 385 amino acid polypeptide with a molecular mass of 42,585 Da that has high sequence similarity (60-70% identity) to plant polyketide synthases. It catalyzes the first step in the cannabinoids biosynthetic pathway. The preferred substrate is hexanoyl-CoA, but accepts also CoA esters with C4 to C8 aliphatic side chains. The olivetolic acid production is promoted when acting in concert with olivetolic acid cyclase (OAC). Taura F. et al. 2009. FEBS Letters 583: 2061-6. The nucleotide sequence of the gene encoding olivetol synthase is found in GenBank under accession number AB 164375 whereas the polypeptide under the accession number BAG14339.

The olivetolic acid cyclase (OAC) is a polyketide cyclase constituted by 101 amino acids (12KDa) which functions in concert with OLS/TKS, as shown in Figure 1, to form olivetolic acid. It requires the presence of OLS to produce olivetolic acid. The *Cannabis sativa* olivetolic acid cyclase accession number is AFN42527.1 (Gagne S.J. et al. 2012. Proc. Natl. Acad. Sci. U.S.A. 109:12811-12816).

CsPT1, is a geranylpyrophosphate olivetolate geranyltransferase, active in the cannabinoid biosynthesis step of prenylation of olivetolic acid to form cannabigerolic acid (CBGA). It is a polypeptide of 395 amino acids. The protein possessed eight transmembrane domains, a predicted chloroplast transit peptide and a prenyl diphosphate binding motif, characteristic of aromatic prenyltransferases.

The cannabidiolic-acid (CBDA) synthase is a 544 aa enzyme (Molecular Mass 62,237 Da) that catalyzes oxidative cyclization of cannabigerolic-acid into CBDA, the dominant cannabinoid constituent of the fiber-type. Cannabis sativa. Cannabidiolic-acid (CBDA) synthase accession number is A6P6V9-1. Taura F. et al. 2007. FEBS Lett. 581:2929-34.

The tetrahydrocannabinolic acid synthase is a 545 aa enzyme (molecular mass 61,927 Da) that catalyzes the oxidative cyclization of the monoterpene moiety in cannabigerolic acid (CBGA), producing Δ(9)-tetrahydrocannabinolate (THCA). The enzyme can also use cannabinerolic acid as substrate, but not cannabigerol or cannabinerol. Sirikantaramas S. et al. 2004. J. Biol. Chem. 279:39767-39774. (Accession number Q8GTB6).

As above described and depicted in the scheme of figure 1, the synthesis of the main cannabinoids requires a combined intervention of different enzymes. Downstream to the first three common step of the synthesis, depending upon the activation of CBDA or THCA synthase, the production of CBD or □⁹THC can be addressed and maximized.

Thereby, in a particularly preferred embodiment of the invention the DNA bacterial expression vector carries the four *Cannabis sativa* genes catalyzing the reaction of biosynthetic pathway leading to the production of cannabidiol:
(i) gene coding for olivetol synthase (OLS) SEQ ID NO: 1,
(ii) gene coding for olivetolic acid cyclase (OAC) SEQ ID NO: 2,
(iii) gene coding for aromatic prenyltransferase (CsPT1) SEQ ID NO: 3,
(iv) gene coding for cannabidiolic acid synthase (CAS) SEQ ID NO: 4.

The probiotic *Lactobacillus Paracasei* subsp. Paracasei F19 transformed with said nucleic acid molecule, for brevity indicated as LP OAC-CAS-OLS-CsPT1+, is able to produce cannabidiol.

In another particularly preferred embodiment of the invention the DNA bacterial expression vector carries the four *Cannabis sativa* genes catalyzing the reaction of biosynthetic pathway leading to the production of Δ⁹-tetrahydrocannabinolic acid:
(i) gene coding for olivetol synthase (OLS) SEQ ID NO: 1,
(ii) gene coding for olivetolic acid cyclase (OAC) SEQ ID NO: 2,
(iii) gene coding for aromatic prenyltransferase (CsPT1) SEQ ID NO: 3,
(iv) gene coding for tetrahydrocannabinolic acid synthase (THCAS) SEQ ID NO: 5.

The insertion of the sequence coding for THC acid synthase, instead of CBDA synthase, enables the probiotic *Lactobacillus Paracasei* subsp. Paracasei F19 transformed with the nucleic acid molecule of this embodiment, for brevity indicated as LP OAC-CAS-OLS-THCAS+, to produce Δ⁹-tetrahydrocannabinolic acid.

According to the invention the capability to orientate the yield toward cannabidiol or THC acid is obtained by the availability of CBD or THCA synthase.

In theory, using the same molecular approach by the differential insertion of the sequence coding for last enzyme of the cannabinoids biosynthesis pathway in the nucleic vector, it is possible to orientate the production of the specific cannabinoid of the probiotic *Lactobacillus Paracasei* subsp. Paracasei F19 also toward the production of other cannabinoids which may have minor or differential therapeutic activities. Further investigation is currently on going by the Applicant. For example, by the insertion of the sequence coding for cannabichromenic acid (CBCA) synthase instead of THCA synthase or CBDA synthase in the vector could lead to an increased level of cannabichromenic acid and cannabichromene.

According to the invention the DNA bacterial expression vector used is the pNIC-BASY vector that through DNA recombinant technology includes the four above mentioned nucleic acid sequences coding for the enzymes involved in the cannabinoid biosynthesis pathways. The inclusion of the four nucleic acid sequences is carried out by four sequentially cloning steps as described in the following experimental section. The final recombinant product has been used to genetically modify the Lactobacillus Paracasei subsp. Paracasei F19 to express the enzymes involved in the cannabinoid biosynthesis pathway.

In a first aspect the invention provides a method for producing therapeutic cannabinoids characterized by administering to an host the probiotic Lactobacillus Paracasei subsp. Paracasei F19, genetically modified to produce and secrete cannabinoids from *Cannabis sativa,* in association with caproic acid, able to establish the enzymatic reactions of the biosynthetic pathway leading to the production of cannabinoids directly *in situ* in said host.

Object of the invention is also the composition comprising Lactobacillus Paracasei subsp. Paracasei F19, carrying four genes from *Cannabis sativa* selected from the group of genes coding for: olivetolic acid cyclase (OAC), cannabidiolic acid synthase (CAS), olivetol synthase (OLS), and aromatic prenyltransferase (CsPT1), or THC acid synthase (THCAS) associated with a dietary supplement of caproic acid, or salts and esters thereof, that allows to obtain high levels of intestinal cannabidiol for the use in the treatment of the gut inflammatory disorders.

In one embodiment of the invention the administration of a composition comprising the transformed Lactobacillus Paracasei subsp. Paracasei F19 LP OAC-CAS-OLS-CsPT1+, enables the consumer to have an increased level of the enzymes involved in the cannabidiol biosynthesis pathway which results in increased production of cannabidiol compounds such as in the gut.

In another embodiment of the invention the administration of a composition comprising the transformed Lactobacillus Paracasei subsp. Paracasei F19 LP OAC-CAS-OLS-THCAS+, enables the consumer to have an increased level of the enzymes involved in the Δ⁹-tetrahydrocannabinolic acid biosynthesis pathway which results in increased production of Δ⁹-tetrahydrocannabinolic acid compounds such as in the gut.

Furthermore, under an external supply of caproic acid (or hexanoic acid), or salts and esters thereof, the multiple engineered probiotic is able to significantly increase the amount of the cannabinoid, either cannabidiol or Δ⁹ THC acid, in vivo in the gut of the consumer.

Caproic acid is the carboxylic acid derived from hexane with the general formula: C₅H₁₁COOH. It is a fatty acid naturally found in various animal fats and oils. It is a colorless oily liquid with an odor that is fatty, cheesy, waxy, and like that of goats.

According to the present invention the consumer is a subject, preferably human and non-human primates, and any other organism, that can benefit from the administration of the multiple engineered probiotic producing a significant amount of cannabidiol, or THCA, *in vivo* in the gut, increased with respect to the subject who has not assumed the engineered probiotic. This subject can also be referred to as the patient, individual, subject, host, user, and organism. In a particularly preferred embodiment of the invention the recipient subject is a human being.

Namely, the organism, that can benefit from the administration of the multiple engineered probiotic producing in the gut a significant amount of cannabidiol, or Δ⁹-tetrahydrocannabinolic acid, *in vivo,* is a patient suffering from inflammatory bowel diseases such as ulcerative colitis, and Crohn's disease, as well as other chronic inflammation status of the mucosa-related diseases and colon cancer.

Therefore, the present invention provides a method to treat inflammatory bowel diseases such as ulcerative colitis, and Crohn's disease, as well as other chronic inflammation status of the mucosa-related diseases and colon cancer. With the term "to treat" it is intended to mean herewith curing, combating, ameliorating, or preventing a disease, disorder, or injury, or relieving the main symptoms related to the pathologic situation.

The method according to the invention involves the administration of a composition which comprises the bacterium Lactobacillus Paracasei subsp paracasei F19 genetically modified to achieve the overproduction of cannabidiol, or Δ⁹-tetrahydrocannabinolic acid, caproic acid or salts and esters thereof, any further elements nutraceuticals, and a vehicle edible, appropriate for the oral administration of the composition.

In a particularly preferred embodiment the engineered probiotic is freeze dried in amounts ranging between 10-30x10¹¹ and 10-30x10¹² cfu (colony forming units) per gram of the composition.

The engineered bacteria according to the invention is part of a probiotic/nutraceutical composition, administered according to the method and comprising 0.001 to 1µg of caproic acid, or salts thereof, per g of preparation, preferably the probiotic/nutraceutical composition is comprising 0.003 to 0.3 µg of caproic acid per g of preparation, even more preferably it comprises 0.3 µg of caproic acid per g of preparation.

The nutraceutical composition administered by the method of the invention may further comprise dietary fiber, maltodextrin, glucoligosaccharides, fructooligosaccharides (GOS/FOS), prebiotics, pectin, inulin, amino acids, minerals and vitamins, especially vitamin C, soy protein or corn, antioxidant agents, commonly used in food compositions in an amount variable in the final nutraceutical composition.

In some embodiments the composition comprising the LP OAC-CAS-OLS-CsPT1+, or LP OAC-CAS-OLS-THCAS+, according to the invention is provided in an edible product suitable for oral administration. Examples of edible product suitable for oral administration include, without limitation: diet bars, beverages, juices, milk, functional beverages, beverages containing dietary fiber, food, yogurt, candy, gum, capsules, granular powder, and tablets.

The dosing regimen of the composition in support of the method according to the invention is defined by the physician according to the real needs of the recipient subject of the treatment. This need is determined by clinical and biochemical analysis which the subject is subjected to before starting treatment and at regular intervals during treatment to verify the effectiveness of the method, the good tolerability by the subject and the lack of effects side.

The amount of caproic acid, or salts and esters thereof, to be daily administered to the patient with the formulation ranges from 0.3 to 0.003 µg per g of composition, preferably from 0.3 to 0.03 µg per g of preparation.

The dosing regimen can be changed and optimized according to the clinical effectiveness in the subject. In particular, for inflammatory bowel the disease course is assessed through evaluation of clinical parameters, such as reduction of diarrhea and increased consistency of stool, pain reduction and normalization of body temperature, and biochemical parameters, such as reduced leukocytosis, and fecal markers such as calprotectin which can be useful aids in distinguishing inflammatory disorders from non-inflammatory gastrointestinal conditions. For functional gastrointestinal disorders the reduction of the severity of specific symptoms and reduction of abdominal pain are evaluated.

The composition according to the invention provides a probiotic-nutraceutical integration devoted to develop an innovative methodology to approach a specific pathological condition not only in the gut, but also in other body compartments, through the development of bacterial cells expressing the whole machinery to produce the natural and non-psychotropic cannabinoid compound, by the sequential insertion of the following genes OAC, CAS, OLS and CsPT1, or OAC, CAS, OLS and THCAS, responsible for CBD, or THCA synthesis in *Lactobacillus paracasei* F19. Under the exogenous boost of caproic acid, or salts and esters thereof, to start the whole process deriving from Hexanoyl CoA (see Fig. 1), the synthesis of CBD, or Δ⁹-tetrahydrocannabinolic acid in the gut is addressed.

This represents an efficient and powerful non psychotropic cannabinoid that exerts a plethora of antinflammatory, anticancer and immunoregolative functions. Very interestingly, the quadruple gene insertion OAC-CAS-OLS-CsPT1⁺, or OAC-CAS-OLS-THCAS⁺, in *Lactobacillus paracasei* F19, associated with a very slight support of caproic acid might open the way to a novel approach that may integrate or substitute traditional pharmacological treatments for a wide range of disease, ranging from gastrointestinal inflammatory diseases to a potential chemo-preventive strategy to counteract inflammatory-associated colorectal cancer, one of the most serious cancer form in humans. In addition, given the well-known effect of cannabinoids as a non-psychoactive analgesic compounds, it would be the ideal strategy to treat the pain in different human pathologies such as cancer, neuropathies and autoimmune diseases. These pathological conditions have a large epidemiological impact associated with their high socio-economic support in terms of co-morbidity, sanitary costs and hospitalization. This feature virtually opens a new medical field whereas a possible introduction of the here-described methodology might have an extraordinary impact also towards a large group of pathological conditions other than bowel inflammatory conditions.

Furthermore, the potential role of cannabinoids, and in particular of CBD, in the beneficial treatment of AD neuropathological features, both *in vitro* and *in vivo,* has been largely suggested in the huge literature available in the field. In order to explore the possibility that the administration of the combination of LP OAC-CAS-OLS-CsPT1⁺, or LP OAC-CAS-OLS-THCAS⁺, and caproic acid (0.3 µg/ml) may induce the production of the relative cannabinoid *in vivo* in a mouse model of Alzheimer's disease, as paradigm of neurodegenerative disease model.

The experimental data from the study carried out by the Applicant also have shown that therapeutic compounds produced by the probiotic bacteria according to the invention can be delivered to tissues beyond the intestinal tract. In fact, it has been found that LP OAC-CAS-OLS-CsPT1⁺, or LP OAC-CAS-OLS-THCAS⁺ administration primarily increased CBD or THCA levels in the colon, consistent with the predominant localization of the probiotic organism in the colon, but also a significant elevation of CBD, or THCA, after sustained LP OAC-CAS-OLS-CsPT1⁺, or LP OAC-CAS-OLS-THCAS⁺ administration, suggesting that cannabinoids absorbed from the colon enters the portal circulation. Thus, our results have demonstrated that the genetically modified bacteria according to the invention, are capable of producing sufficient amounts of secreted cannabonoids to induce significant therapeutic effects, even when the target organ is not the intestinal tract. Cannabinoids, introduced in the organism by genetically modified *Lactobacillus paracasei* F19 as therapeutic molecules are well absorbed, but such slowly metabolized, to be targeted even to more distal organs such as the brain.

Results have shown that the invention provides an efficient method to administrate therapeutic cannabinoids, preferably CBD and THCA, overcoming most of the drawbacks related to the administration of therapeutically effective amount of cannabinoids as such, or medicaments based on cannabis extracts, i.e. great variability of action, difficulties in optimizing the dosage, systemic side effects, psycotropicity, ecc..., to subjects in the need thereof.

According to the invention said subjects are affected by bowel inflammatory conditions, colon carcinoma and neurodegenerative condition such as Alzheimer's Disease patients.

Other aspects, advantages and modifications within the scope of the invention will be apparent to those skilled in the relevant field of invention.

Only for descriptive and not limiting purposes an examples of the nutraceutical composition of the present invention is presented below.

### EXAMPLE 1 - Formulation

| **Ingredient** | **Amount per 100 g of product** | **Daily intake** |
|---|---|---|
| *L. paracasei F19* OAC-CAS-OLS-CsPT1+ | 1200-2400*10¹¹ (cfu) | 12-24*10¹¹ (cfu) |
| Caproic acid | 30 µg | 0.3 µg |

### EXAMPLE 2 - Amplification and cloning of cDNA sequences of interest and preparation of pNIC-BASY OAC-CAS-OLS-CsPT1+ construct.

1. cDNA sequences of the genes of interest: olivetol synthase (OLS), olivetolic acid cyclase (OAC), aromatic prenyltransferase (CsPT1), cannabidiolic acid synthase (CAS) were obtained by RT PCR from total RNA extracted from Trichome of *Cannabis sativa* using the primers listed below. OAC-FW:5’-TACTTCCAATCCATGGCAGTGAAGCATTTGATTGTATTG-3’; 
OACRV:5’-
TATCCACCTTTACTGTCACTTTCGTGGTGTGTAGTCAAAAATG-3’;
CAS-FW: 5’-TACTTCCAATCCATGAAGTGCTCAACATTCTCCTTTTGG-3’;
CAS-RV: 5’-
TATCCACCTTTACTGTCAATGACGATGCCGTGGAAGAGGTG-3’;
OLS-FW: 5’-TACTTCCAATCCATGAATCATCTTCGTGCTGAGG-3’;
OLS-RV: 5’-
TATCCACCTTTACTGTCAATATTTGATGGGAACACTACGCAC-3’;
CsPT1-FW: 5’-TACTTCCAATCCATGGGACTCTCATCAGTTTGTACC-3’;
CsPT1-RV: 5’-
TATCCACCTTTACTGTCATATGAAAACATATACTAAATATTCAGCATAA
TAAAGC3’.
 Each primer pair carries flanking sequences specific for sequence around the *Bsa*I site of pNIC-BASY vector which were used for overlapping in In-Fusion method from Clontech in the next step of cloning procedure.
2. The pNIC-BASY vector was digested with *Bsa*I and each PCR product from step one were inserted into the cutting site using In-Fusion method from Clontech, resulting four constructs: pNIC-BASY-OAC, pNIC-BASY-CAS, pNIC-BASY-OLS, and pNIC-BASY-CsPT1.
3. The inserts of the four constructs were assembled in one pNIC-BASY vector performing the following cloning strategy. Using pNIC-BASY-CAS as backbone, the gene expression components of pNIC-BASY-OAC including araBAD promoter, and OsmY-TEV-OAC fusion protein sequence (OsmY is bacterial membrane protein, and TEV is protease site used to release OAC from OsmY once it is secreted from the bacteria cell) were PCR amplified and cloned into the pNIC-BASY-CAS backbone at *Age*I restriction site. Then, araBAD promoter and OsmY-TEV-OLS fragments are PCR amplified from pNIC-BASY-OLS, and inserted into pNIC-BASY-OAC-CAS at *Eco*RI/*Sac*I restriction sites. Finally, araBAD promoter and OsmY-TEV-CsPT1 fragments are cloned into pNIC-BASY-OAC-CAS-OLS at *Sac*I/*Not*I restriction sites to get the construct of pNIC-BASY-OAC-CAS-OLS-CsPT1. The PCR primers used are listed below:
   OsmY-OAC-FW-*Age*I: 5'-CCAAACCGGTTAACAAAGCGGGACCAAAGC-3';
   OsmY-OAC-RV-*Age*I: 5'-ATCGACCGGTCCACCTTTACTGTCACTTTCGT-3';
   OsmY-OLS-FW-*Eco*RI: 5'-ATCCGAATTCGGGACCAAAGCCATGACAAA-3';
   OsmY-OLS-RV-*Sac*I: 5'-GACGGAGCTCTCCGTATCCACCTTTACTGTCA-3';
   OsmY-CsPT1-FW-*Sac*I: 5'-ATTCGAGCTCTAACAAAGCGGGACCAAAGC-3';
   OsmY-CsPT1-RV-*Not*I: 5'-GAGTGCGGCCGCTCCGTATCCACCTTTACTGTCA-3'.

All cloning procedures were verified by sequencing.

### EXAMPLE 3 - Expression of recombinant pNIC-BASY-OAC-CAS-OLS-CsPT1+ in Lactobacillus paracasei F19.

Transformation of pNIC-BASY-OAC-CAS-OLS-CsPT1+ vector into *Lactobacillus Paracasei* F19 were performed using the standard heat shock method.

Bacteria LP-OAC-CAS-OLS-CsPT1⁺ and LP-OAC-CAS-OLS-CsPT1⁻ carrying the empty pNIC-BASY plasmid as negative control (LP-empty vector) were cultured in Luria Bertani (LB) or LB-agar (1.2%) medium at 30°C or 37°C, respectively, and exposed to increasing concentrations of caproic acid (10⁻⁹-10⁻⁷ M).

After different time-points intervals (12-24 hrs) the content of released CBD in the bacterial supernatant was measured. Bacterial supernatant aliquots were analyzed for the quantification of CBD released by liquid chromatography coupled to tandem mass spectrometry (LC-MS/MS) using a 325 MS LC/MS using a 325-MS LC/MS Triple Quadrupole Mass Spectrometer (Agilent Technologies Italia, Cernusco s/N, Italy).

### Results

Results of the study conducted to confirm the ability of LP OAC-CAS-OLS-CsPT1+ (10¹¹ cfu) to produce and release in bacterial medium significant amount of CBD in the presence of caproic acid (10⁻⁹-10⁻⁷M) have demonstrated in 12-24 hrs time-course experiments that CBD-release significantly increases over the time and it is dependent on the concentration of the caproic acid added to the culture medium. The release of CBD in bacterial medium was measured by liquid chromatography coupled to tandem mass spectrometry (LC-MS/MS) using a 325-MS LC/MS Triple Quadrupole Mass Spectrometer (Agilent Technologies Italia, Cernusco s/N,Italy). The production of CBD by LP-OAC-CAS-OLS-CsPT1⁺ significantly increased over the time, starting at 12 hrs and peaking its highest level at 24 hrs post incubation. (***P<0.001 vs. vehicle). The LP empty vector was unable to produce any significant amount of CBD, as expected. (Figure 2).

### Example 4 - Evaluation of the ability of LP OAC-CAS-OLS-CsPT1+ to produce and release CBD in a mouse models of acute and/or chronic colitis

The effect of 5x10¹⁰ cfu of both LP OAC-CAS-OLS-CsPT1⁺ or saline vehicle as control by gavage was tested in an acute colitis protocol in mice exposed to dextran sulphate sodium (DSS) for 7 days.

*Animals:* Six-weeks-old male CD-1 mice (25 to 35 g; Harlan Laboratories, Udine, Italy) or IL-10 KO mice from Taconic were used for the experiments. Animals were randomly divided into five groups (n = 10 each) and subjected to the treatment according to the experimental plan. In CD-1 mice, experimental colitis was induced by administrating DSS (4% w/v, MW 36,000 to 50,000) in drinking water for six consecutive days.

The combined effect of a caproic acid enriched diet (0.003-0.3 µg/ml daily supplement) in the animals exposed to the LP OAC-CAS-OLS-CsPT1⁺ gavage, was assayed in terms of amelioration of DAI (Disease Activity Index). The degree of disease severity has been expressed by the DAI score, based on the evaluation of different parameters characterizing the experimental colitis, both in its induction phase and progression. Body weight, presence of gross blood in the feces and stool consistency were recorded daily (from day 0 to 7 or 0-21 depending the model) by an observer blinded to the treatment. The DAI was determined by scoring changes in: weight loss (0 = none; 1 = 1 to 5%; 2 = 5 to 10%; 3 = 10 to 20%; 4 = >20%); stool consistency (0 = normal; 2 = loose; 4 = diarrhea) and rectal bleeding (0 = normal; 2 = occult bleeding; 4 = gross bleeding). At the end of the experiment, mice were sacrificed and colons and spleens were isolated to measure the length and weight of colon and spleen, respectively.

Post post-mortem evaluation of histological inflammation parameters such as lymphocyte/macrophage colonization of the colon, biochemical/molecular release of mieloperoxidase (MPO), Nitric oxide (NO) and prostaglandin E2 (PGE2) and/or related proteins (iNOS, COX-2) was also performed by standard procedures.

*Preparation of cytosolic extracts and Western blot analysis.* Removed colonic tissues were processed for western blot analysis. Briefly, after homogenization in ice-cold hypotonic lysis buffer, protein concentration was determined using Bio-Rad protein assay kit (Bio-Rad, Milan, Italy). Analysis of cyclooxigenase (COX)-2, iNOS, β-actin protein expression was performed on total protein fractions of homogenates. Equivalent amounts (50 µg) of each homogenate underwent electrophoresis through a polyacrilamide minigel. Proteins were then transferred onto nitrocellulose membrane that were saturated by incubation with 10% nonfat dry milk in 1× PBS overnight at 4°C and then incubated with either mouse anti-iNOS (1:2000 v/v, BD Biosciences, Milan, Italy), rabbit anti-COX-2 (1:250 v/v, BD Biosciences), or mouse anti-β-actin (1:1,000 v/v, Santa Cruz Biotechnology) for 2 hrs at room temperature (RT). Membranes were then incubated with anti-mouse or anti-rabbit immunoglobulins coupled to peroxidase (1:2000 v/v, Dako, Milan, Italy). Immune complexes were revealed by using enhanced chemiluminescence detection reagents (Amersham Biosciences, Milan, Italy) and exposed to Kodak X-Omat film (Eastman Kodak Co., Rochester, NY, USA OK). Protein bands were then scanned and densitometrically analyzed with a GS-700 imaging densitometer.

*NO quantification.* NO was measured as nitrite (NO²⁻) accumulation in mice and human biopsies colon homogenates by a spectrophotometer assay based on the Griess reaction.29 Briefly, Griess reagent (1% sulphanilamide, 0.1% naphthylethylenediamine in H3PO4) was added to an equal volume of supernatant and the absorbance was measured at 550 nm. Nitrite concentration (nM) was thus determined using a standard curve of NaNO2.

*Enzyme-linked immunosorbent assay for PGE₂.* Enzyme-linked immunosorbent assay (ELISA) for PGE₂ (R&D Systems, Minneapolis, Minnesota, USA) was carried out on plasma of mice and on human biopsies supernatants according to the manufacturer's protocol. Absorbance was measured on a microtitre plate reader. PGE2 levels were determined using standard curves method.

### Example 5 - Evaluation of the antiinflammatory effects on human intestinal biopsies from patients with ulcerative colitis

The effect of LP-OAC-CAS-OLS-CsPT1⁺ was tested on human intestinal biopsies from patients with ulcerative colitis.

The experimental group comprised 8 patients diagnosed with UC (5 women; mean age 47 years) and eight control undergoing colonoscopy for colon cancer screening, (2 women; mean age 50 years). All persons received and signed an informed consent. All procedures were approved by the ethical committee of the University of Naples Federico II. In all individuals, four mucosal biopsies from the rectosigmoid region were collected and cultured in FBS-supplemented Dulbecco Modified Eagle's Medium (DMEM) at 37°C in 5% CO₂/95% air. All biopsies were cultured, for 24-48 hrs, with or without (10¹¹cfu) LP-OAC-CAS-OLS-CsPT1⁺ or equal amount of LP empty vector in the presence of caproic acid (0.003-0.3µg/ml). Biopsies were then homogenised and analysed by western blot as described below. In the same experimental conditions, some samples were fixed in PFA and used for immunohistochemical analysis.

### Results

As shown in Figure 3, results indicate that the administration of LP-OAC-CAS-OLS-CsPT1⁺ (10¹¹ cfu), combined to daily administration of caproic acid (0.003-0.3µg/ml), induced a significant clinical improvement of DSS-induced colitis, with a significant reduction of DAI score parameters (disease course, colon shortening and splenomegaly). In addition, in mice treated with LP-OAC-CAS-OLS-CsPT1⁺ a significant reduction of iNOS and COX-2 expression was observed, together with a marked decrease of NO², PGE₂ and MPO; similarly macrophages infiltration was found to be significantly reduced.

Furthermore, the antinflammatory effects of LP-OAC-CAS-OLS-CsPT1⁺ in combination with caproic acid in human intestinal biopsies from patients with ulcerative colitis paralleled the results obtained in mice and yield to a significant decrease of NO and PGE2 release, iNOS and COX-2 protein expression, MPO levels, respectively; these effects were absent when the biopsies were incubated with the LP-empty vector even if combined with the highest dose of caproic acid.

Figure 3 shows the effects of LP-OAC-CAS-OLS-CsPT1⁺ combined with caproic acid on colon inflammation. In particular, in Figure 3A the ameliorative effect produced by LP-OAC-CAS-OLS-CsPT1⁺ associated to (0.003-0.3µg/ml) daily administration or caproic acid gavage on DSS induced colitis in mice is depicted. The LP-OAC-CAS-OLS-CsPT1⁺/caproic acid association reduced the disease activity index (DAI) severity, contained colon shortening and reduced splenomegaly induced by the colitis.

As consequence of symptomatic relief, LP-OAC-CAS-OLS-CsPT1⁺ associated to (0.003-0.3 µg/ml) caproic acid reduced both iNOS and COX-2 protein expression in mice colon exposed to DSS (figure 3 B); and such activity was fully paralleled to iNOS and COX-2 (Figure 3C and 3D) as well as NO₂ (Figure 3E) and PGE₂ (Figure 3F) release decrease in UC deriving organotypic cultured biopsies. The effect produced by LP-OAC-CAS-OLS-CsPT1⁺ associated to (0.003-0.3µg/ml) administration on MPO release in both mice and human colitic samples, as confirmative of overall amelioration of pro-inflammatory scenario in tested experimental conditions is shown in Figure 3G (***P<0.001; **P<0.01; *P<0.05 vs controls;°°°P<0.001; °°P<0.01; °P<0.05 vs experimental colitic group).

### Example 6 - Evaluation of LP OAC-CAS-OLS-CsPT1+ in association with and caproic acid in IL-10 KO chronic mice colitis model

The effectiveness of LP-OAC-CAS-OLS-CsPT1⁺ with a supply of caproic acid was evaluated in an animal model of chronic colitis using the IL-10 KO mice that genetically develop a chronic colitis similar to human chronic colitis. Results have demonstrated that the administration of LP-OAC-CAS-OLS-CsPT1+ and caproic acid significantly improved the colon inflammation condition in this mice colitis model. Also in this chronic colitis model, the levels of MPO, NO and PGE2 and inflammatory proteins (iNOS, COX-2) were significantly reduced. In addition, as the experimental observation was carried out for 21 days, the results support the long-term efficacy of the composition in a model of chronic colitis and extend the therapeutic significance to a chronic context beyond the acute phase efficacy. In Figure 4, the beneficial effects of the invention are summarized.

### Results

The beneficial effect produced by LP-OAC-CAS-OLS-CsPT1⁺ associated to (0.003-0.3µg/ml) daily administration of caproic acid by gavage on IL-10 KO mice at 21 days is shown in Figure 4. The LP-OAC-CAS-OLS-CsPT1⁺/caproic acid association reduced the disease activity index (DAI) severity, contained colon shortening and reduced splenomegaly induced by the colitis (Figure 4A). LP-OAC-CAS-OLS-CsPT1⁺ associated to (0.003-0.3µg/ml) reduced NO₂ release (Figure 4B) and both iNOS protein expression (Figure 4C) and COX-2 (Figure 4F) protein expression in mice colitic colon. Also PGE2 release decreased in the tissues (Figure 4E) and the overall amelioration of pro-inflammatory scenario in tested experimental conditions LP-OAC-CAS-OLS-CsPT1⁺ associated to (0.003-0.3µg/ml) administration was confirmed by MPO release data in IL-10 KO mice (Figure 4D).(***P<0.001; **P<0.01; *P<0.05 vs controls;°°°P<0.001; °°P<0.01; °P<0.05 vs experimental colitic group)

### Example 7 - Evaluation of the anticancer effect of LP OAC-CAS-OLS-CsPT1+ in association with caproic acid administration in a mouse colon carcinoma model and in human colon carcinoma cells

The putative cancer-preventive action of the CBD-releasing probiotic in a mice model of sporadic colon cancer, was carried out in Apc^{min/+} mice.

*Animals* Male APCMin/+ mice obtained from The Jackson Laboratory (Bar Harbor, USA) were crossed with wild-type C57BL/6 female mice to generate APCMin/+ mice. A total of 20 APCMin/+ mice (age, 4 weeks) were randomly divided into three groups respectively receiving saline solution (vehicle group), LP oac/cas/ols/CsPT1+ and caproic acid 0.3µg/ml, and LP empty vector and caproic acid 0.3µg/ml daily for 4 weeks daily.

Following administration through gavage with 5 x 10¹⁰ cfu of LP OAC-CAS-OLS-CsPT1⁺, or saline as control, the incidence of colon carcinoma in mice was assessed. Eight-to-ten weeks old mice were daily administered with 5 x 10¹⁰ cfu of LP OAC-CAS-OLS-CsPT1⁺, and the incidence of colon cancer was compared to animals administered with empty vehicle (control). The experimental plan was carried for the entire life expectancy of the animals. At the end of the protocol, all the animals were tested for cancer development screening using a multidisciplinary approach, including immunohistological, bioenzymatic, biochemical marker detection related to colon cancer detection and staging.

*Tumor analysis.* After experimental treatments, following CO₂ euthanasia at 80 days of age, the intestinal tract was removed and flushed with ice-cold phosphate-buffered saline and immediately fixed in 10% neutral buffered formalin for 48 hours. The small intestine was measured into three equal sections for identifying polyp location. Polyps were counted blindly under a dissecting microscope and categorized as small (1-3 mm), or large (>3 mm). By histological analysis, aberrant foci, number of polyps and tumors were counted for the different groups. Relative results are shown in Figure 5A-D.

To confirm the chemo-preventive action of cannabidiol in the experimental conditions, a study on the putative pro-apoptotic and anticancer activity exerted by LP OAC-CAS-OLS-CsPT1+ probiotic was tested in vitro in human colon carcinoma cells. CBD release in human colon carcinoma cell line was measured at the different time points.

*Caco-2 cell colture.* Caco-2 cells were cultured in Dulbecco's Modified Eagle's Medium (DMEM) supplemented with 5% Foetal Bovine Serum (FBS), 2 mM glutamine, 100 U/mL penicillin, 100 µg/mL streptomycin at 37 °C in 5% CO₂/95% air. At confluence cells were washed three times with Phosphate Buffer Saline (PBS), detached with tripsyn/EDTA, plated in 10 cm diameter Petri dishes and left to adhere for 24 hrs. Thereafter, DMEM was replaced with fresh medium and cells were treated for 96 hrs with 10¹¹ cfu LP OAC-CAS-OLS-CsPT1⁺, with caproic acid 0.003-0.3µg/ml or LP empty vector with caproic acid 0.003-0.3µg/ml.

The pro-apoptotic effect in Caco-2 cell cultured in the presence of LP OAC-CAS-OLS-CsPT1+ or LP empty vector was tested by immunoblot analysis of pro-apoptotic proteins level (Bax), as well as by rate of cells proliferation (MTT).

*Immunoblot of Bax protein.* Briefly, after homogenization in ice-cold hypotonic lysis buffer, protein concentration was determined using Bio-Rad protein assay kit (Bio-Rad, Milan, Italy). Analysis of Bax and β-actin protein expression was performed on total protein fractions of homogenates. Equivalent amounts (30 µg) of each homogenate underwent electrophoresis through a polyacrilamide minigel. Proteins were then transferred onto nitrocellulose membrane that was saturated by incubation with 10% nonfat dry milk in 1× PBS overnight at 4°C and then incubated with either mouse anti-Bax (1:200 v/v, Neo-Marker, Milan, Italy), or mouse anti-β-actin (1:1,000 v/v, Santa Cruz Biotechnology) for 2 hrs at room temperature (RT). Membranes were then incubated with anti-mouse or anti-rabbit immunoglobulins coupled to peroxidase (1:2000 v/v, Dako, Milan, Italy). Immune complexes were revealed by using enhanced chemiluminescence detection reagents (Amersham Biosciences, Milan, Italy) and exposed to Kodak X-Omat film (Eastman Kodak Co., Rochester, NY, USA OK). Protein bands were then scanned and densitometrically analyzed with a GS-700 imaging densitometer.

*MTT Proliferation and survival assays.* Cell proliferation was evaluated by 3-[4,5-dimethylthiazol-2-yl]-2,5 diphenyltetrazolium bromide (MTT) assay. In brief, Caco-2 cells were placed in 96-well plates and allowed to adhere for 2 hours. Thereafter, DMEM was replaced with fresh medium and the cells were treated as described. After 96 hours, 25 µl MTT (5mg/ml in DMEM) were added to the cells and incubated for additional 3 hours at 37°C. After this time point, the cells were lysed and the dark blue crystals solubilized with 125 µl of a solution containing 50% (N,N-dimethylformamide, 20% (w/v) sodium dodecylsulphate (pH 4.5). The optical density (OD) of each well was measured with a Perkin-Elmer (Waltham, MA, USA) microplate spectrophotometer equipped with a 620 nm filter. Cell viability in response to pentamidine was calculated as % cell viability (OD treated/OC control) X 100.

### Results

In our experimental conditions LP OAC-CAS-OLS-CsPT1⁺ and 0.3 µg/ml caproic acid supplement was daily given by gavage to Apc^{min/+} mice which revealed a significantly increased survival both versus tumour group and LP empty vector with caproic acid 0.3µg/ml supplement (Figure 5 A). Post mortem analysis revealed that LP OAC-CAS-OLS-CsPT1⁺ caproic acid 0.3 µg/ml supplement treatment significantly reduced aberrant crypt foci (ACF), polyps and tumors in the colon both versus tumour group and LP empty vector + caproic acid 0.3µg/ml supplement (Figure 5B, C, D). (*P<0.05 vs. vehicle treated tumor group).

More in detail, the obtained results indicate that LP OAC-CAS-OLS-CsPT1⁺ supplemented with 0.3 µg/ml caproic acid increase survival of Apc^{min/+} mice (Figure 5 A); such effect was not evident when LP empty vector and 0.3 µg/ml caproic acid was given to the animals. Furthermore, the administration of probiotic LP OAC-CAS-OLS-CsPT1⁺ + caproic acid 0.3 µg/ml supplement reduces aberrant foci number (Figure 5 B), polyps (Figure 5 C) and tumors (Figure 5 D) by microscopy inspection in post mortem colon analysis. No difference were observed using LP empty vector associated to caproic acid 0.3µg/ml treatment versus untreated (vehicle) animals (*p<0.05 vs. vehicle).

Results further demonstrated that 96 hrs incubation of this probiotic combined with caproic acid had an antiproliferative effect in the cell culture, and significantly increased the expression level of the pro-apoptotic protein Bax-2 (Figure 5E and F)(***P<0.001 vs. vehicle). Conversely, when in the culture the LP empty vector or simply caproic were added, even at the highest concentration, no significant effect was observed (Figure 5E and F).

### Example 8 - Neuroprotective effect of the co-administration of LP-OAC-CAS-OLS-CsPT1+ and caproic acid in a mouse model of Alzheimer's disease

In order to explore the possibility that the administration of the combination of LP-OAC-CAS-OLS-CsPT1+ and caproic acid *(*0.3 µg/ml) may induce the production of CBD *in vivo* in a mouse model of Alzheimer's disease, as paradigm of neurodegenerative disease model, and its possible therapeutic activity, the impact of the invention in a triple transgenic mouse model of AD, the 3xTg AD mice model that very similarly resembles the AD features occurring in humans, has been analyzed. The effect of the administration of 5x10¹¹ ufc of LP-OAC-CAS-OLS-CsPT1+ and caproic acid supply (0.3µg/ml) in 3Tg-AD mice for 4 weeks was evaluated. The gavage administration was started when the mice reached the 6^{th} weeks of age. Such age was considered strategically important since it was commonly referred to the appearance of neuropathological features of the disease. 4 weeks post treatments the following parameters were evaluated:
1. the content of CBD released in the hippocampus of 3xTg-AD mice which received LP OAC-CAS-OLS-CsPT1+ and the caproic acid supply (0.3µg/ml);
2. the number of senile amyloid plaques in the frontotemporal cortex of the animals
3. the presence of hippocampal neurodegeneration signs in CA1 area
4. neuroinflammation underlining neurodegeneration in the brain of 3xTg AD mice.

*CBD quantification in mice hippocampus*. The release of CBD in hippocampus has been measured post mortem according to the experimental plans through by liquid chromatography coupled to tandem mass spectrometry (LC-MS/MS) using a 325-MS LC/MS Triple Quadrupole Mass Spectrometer (Agilent Technologies Italia, Cernusco s/N,Italy) as above described.

*Triple transgenic AD mice* (3xTg AD mice) were obtained by JAX (Jackson laboratories). Six weeks animals were randomly divided into five groups, wild type CD-1 mice (n = 10 each) were used as control. Mice received for 4 weeks the treatment with 5x10¹¹ ufc LP OAC-CAS-OLS-CsPT1+ and 0.3µg/ml caproic acid versus LP empty vector+ + caproic acid supply (0.3µg/ml).

*Histological analysis.* At the end of the experimental plan, mice were anesthetized with ketamine and xylazine and perfused with 4% paraformaldehyde in 0.1 mol/L phosphate buffer solution (pH 7.4). Then, the brains were removed and fixed in the similar solution for 24 hrs. Following routine processing in paraffin, serial coronal sections of the brain were cut at 8 µm thickness in a rotary microtome (Leitz, 1512, Germany). Brain sections were stained with Nissl staining, and modified Congo red staining according the respective manufacturer's instruction (Sigma-Aldrich St Luois, MO, USA). Images were then digitalized by NikonEclipse camera connected to the microscope, and taken at 20 or 50X magnification.

*Immunoblot analysis of pro-inflammatory proteins in hippocampus*. After brain homogenization in ice-cold hypotonic lysis buffer, protein concentration was determined using Bio-Rad protein assay kit (Bio-Rad, Milan, Italy). Analysis of S100B, GFAP, iNOS, COX-2 and and β-actin protein expression was performed on total protein fractions of homogenates. Equivalent amounts (30 µg) of each homogenate underwent electrophoresis through a polyacrilamide minigel. Proteins were then transferred onto a nitrocellulose membrane saturated by incubation with 10% nonfat dry milk in 1× PBS overnight at 4°C and then incubated with specific antibody for 2 hrs at room temperature (RT). Membranes were then incubated with anti-mouse or anti-rabbit immunoglobulins coupled to peroxidase (1:2000 v/v, Dako, Milan, Italy). Immune complexes were revealed by using enhanced chemiluminescence detection reagents (Amersham Biosciences, Milan, Italy) and exposed to Kodak X-Omat film (Eastman Kodak Co., Rochester, NY, USA OK). Protein bands were then scanned and densitometrically analyzed with a GS-700 imaging densitometer.

### Results

The experimental data have shown that the administration of a composition comprising 5x10¹¹ ufc of LP OAC-CAS-OLS-CsPT1⁺ in association with 0.3µg/ml caproic acid to 3xTg-AD mice for 4 weeks caused a significant release of CBD in the hippocampus of the animals, as compared to their matched internal controls which received LP empty vector and equal amount of caproic acid (Figure 6 A). As shown in Figure 6B, 3xTgAD mice relieved a significant increase of the number of plaques respect to wild type mice, and a marked depression of pyramidal neurons in the CA1 areas (Figure 6 C), both features are indicative of AD-like neurodegeneration. As shown in panels B and C, in 3xTgAD mice, the administration of 5x10¹¹ ufc of LP OAC-CAS-OLS-CsPT1⁺ and 0.3µg/ml caproic acid for 4 weeks, caused a significant reduction of the number of plaques and neuronal loss in the hippocampus (respectively, panel B and C). No significant results were obtained when LP empty vector in presence of caproic acid supply (0.3µg/ml) were administered.

In addition, molecular analysis of reactive gliosis and pro-inflammatory proteins in the hippocampus (GFAP, S100B, iNOS and COX-2) were evaluated. According to the above reported results, all proteins were increased in 3xTgAD; the administration of 5x10¹¹ ufc of LP OAC-CAS-OLS-CsPT1+ with caproic acid (0.3µg/ml) caused a significant decrease of all tested reactive gliosis and pro-inflammatory proteins (Figure 6 D), confirming the antinflammatory/neuroprotective function of this treatment likely dependent on the increased production of CBD in the mice brain. No significant results were obtained when LP empty vector and caproic acid were administrated.

In particular, Figure 6A shows the release of CBD in the brain of 3TgAD mice following 4 weeks treatment with 5x10¹¹ cfu LP OAC-CAS-OLS-CsPT1+ and caproic acid (0.3µg/ml) versus the treatment with LP empty vector and caproic acid supply (*p<0.05 vs LP empty vector). The arrows in Figure 6 B indicate senile plaques of amyloid beta in 3xTgAD mice, the picture shows the effect of the invention against amyloid plaques accumulation in the frontotemporal cortex following 4 weeks treatment. Figure 6C shows the CA1 neuronal loss induction in 3xTgAD mice compared to wt mice. A 4 weeks treatment demonstrated to be able to re-establish CA1 integrity.

Figure 6D shows Western blot analysis of pro-inflammatory proteins in the hippocampus from wt or 3TgAD mice and the antinflammatory effect of 4 weeks administration of 5x10¹¹ cfu LP OAC-CAS-OLS-CsPT1+ and caproic acid (0.3µg/ml) in the areas (***P<0.001 vs vehicle wt; °°° P<0.001; °°P<0.01 vs 3xTg-AD).

### EXAMPLE 9 - Formulation

| **Ingredient** | **Amount per 100 g of product** | **Daily intake** |
|---|---|---|
| *L. paracasei F19* OAC-CAS-OLS-THCAS+ | 1200-2400*10¹² (cfu) | 12-24*10¹² (cfu) |
| Caproic acid | 30 µg | 0-3 µg |

### EXAMPLE 10 - Amplification and cloning of cDNA sequences of interest and preparation of pNIC-BASY OAC-CAS-OLS-THCAS+ construct.

1. cDNA sequence of the gene tetrahydrocannabinolic acid synthase (THCAS) was obtained by RT PCR from total RNA extracted from Trichome of *Cannabis sativa* using the pair of primers:
   THCAS-FW: 5-TACTTCCAATCCATGAATTGCTCAGCATTTTCCTTTTGG-3';
   10THCAS-RV: 5'-TATCCACCTTTACTGTCAATGATGATGCGGTGGAAGAGGTG-3'.

Both primers carry flanking sequences specific for the sequence around the *Bsa*I site of pNIC-BASY vector which were used for overlapping in In-Fusion method from Clontech in the next step of cloning procedure.
2. The pNIC-BASY vector was digested with *Bsa*I restriction enzyme and the PCR products from step one was inserted into the cutting site using In-Fusion method from Clontech, resulting the construct: pNIC-BASY-THCAS.
3. Using pNIC-BASY-OAC-CAS-OLS as backbone, the gene expression component of pNIC-BASY-THCAS including araBAD promoter, and OsmY-TEV-THCAS fusion protein sequence was PCR amplified and cloned into the pNIC-BASY--OAC-CAS-OLS backbone at *Age*I restriction site. Finally, araBAD promoter and OsmY-TEV-THCAS fragments are cloned into pNIC-BASY-OAC-CAS-OLS at *Sac*I/*Not*I restriction sites to get the construct of pNIC-BASY-OAC-CAS-OLS-THCAS. The PCR primers used are listed below:
   OsmY-THCAS-FW-*Sac*I: 5'-TATCCACCTTTACTGTCATATGAAAACATATACTAAATATTCAGCATAA TAAAG -3';
   OsmY-THCAS-RV-*Not*I: 5'-TATCCACCTTTACTGTCATATGAAAACATATACTAAATATTCAGCATAA TAAAGC -3'.

The cloning procedure leads to the generation of the plasmid pNIC-BASY-OAC-CAS-OLS-THCAS.

All cloning procedures were verified by sequencing.

### EXAMPLE 11 - Expression of recombinant pNIC-BASY-OAC-CAS-OLS-THCAS⁺ in Lactobacillus paracasei F19.

Transformation of pNIC-BASY-OAC-CAS-OLS-THCAS⁺ vector into *Lactobacillus Paracasei* F19 were performed using the standard heat shock method.

Bacteria LP-OAC-CAS-OLS-THCAS⁺ and LP-OAC-CAS-OLS-CsPT1⁻ carrying the empty pNIC-BASY plasmid as negative control (empty vector) were cultured in Luria Bertani (LB) or LB-agar (1.2%) medium at 30°C or 37°C, respectively, and exposed to increasing concentrations of caproic acid (10⁻⁹-10⁻⁷ M).

After different time-points intervals (12-24 h) the content of released Δ⁹THC in the bacterial supernatant was measured. Bacterial supernatant aliquots have been analyzed for the quantification of Δ⁹THC released by liquid chromatography coupled to tandem mass spectrometry (LC-MS/MS) using a 325 MS LC/MS using a 325-MS LC/MS Triple Quadrupole Mass Spectrometer (Agilent Technologies Italia, Cernusco s/N, Italy).

### Results

In a 12-24 hrs time-course experiment, the Δ⁹⁻THC release resulted to be significantly increased over the time and dependent on the concentration of the caproic acid added to the culture medium. Figure 7 clearly shows that when LP OAC-CAS-OLC-THCAS+ (10¹¹ cfu) was incubated in the presence of caproic acid (10⁻⁹-10⁻⁷ M) in vitro a significant and dose dependent amount of Δ⁹ THC was released in the bacterial medium; the production started at 12 hrs and peaked at 24 hrs post incubation. (***P<0.001 vs vehicle). The control LP empty vector was unable to produce any significant amount of THCAS, as expected.

## Claims

1. *Lactobacillus Paracasei* subsp paracasei F19 transformed with a nucleotide vector comprising a nucleic acid carrying four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.

2. *Lactobacillus Paracasei* subsp paracasei F19 according to claim 1 **characterized by** a nucleotide vector comprising a nucleic acid carrying the Cannabis sativa genes:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3, and
(iv) coding sequence for cannabidiolic acid synthase (CAS) SEQ ID NO: 4.

3. *Lactobacillus Paracasei* subsp paracasei F19 according to claim 1 **characterized by** a nucleotide vector comprising a nucleic acid carrying the *Cannabis sativa* genes:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3, and
(iv) coding sequence for tetrahydrocannabinolic acid synthase (THCAS) SEQ ID NO: 5.

4. A composition comprising:
*Lactobacillus Paracasei* subsp paracasei F19 transformed with a nucleotide vector comprising a nucleic acid carrying four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
- caproic acids, or salts and esters thereof,
- an ingestible vehicle suitable for oral administration of the composition.

5. The composition according to claim 4 further comprising dietary fiber, maltodextrin, glucoligosaccharides, fructooligosaccharides, prebiotic agents, pectin, inulin, amino acids, mineral salts, vitamins, soy protein, corn protein, antioxidant agents.

6. The composition according to claim 4 wherein the transformed Lactobacillus Paracasei subsp paracasei F19 is in lyophilized form in an amount ranging between 10-30x10¹¹ and 10-30x10¹² cfu per gram of composition.

7. The composition according to claim 4 wherein the caproic acid is present in an amount ranging between from 0.001 to 1 µg per g of composition.

8. The composition according to claim 7 wherein the caproic acid is present in an amount ranging between 0.25 and 0.35 µg per g of composition.

9. Composition according to claims 4 to 8 provided in an edible product.

10. Composition according to claim 9, wherein the edible product is a dietary bar, beverage, juice, milk, functional beverage, beverage comprising dietary fiber, food, yogurt, candy, gum, capsules, granular powder, tablets.

11. A method for producing of therapeutic cannabinoids **characterized by** administering to an host the probiotic Lactobacillus Paracasei subsp. Paracasei F19, genetically modified to produce and secrete cannabinoids from *Cannabis sativa,* in association with caproic acid, able to establish the enzymatic reactions of the biosynthetic pathway leading to the production of cannabinoids directly *in situ* in said host.

12. Method according to claim 1 wherein the therapeutic cannabinoids are cannabidiolic acid (CBDA), cannabidiol (CBD), Δ9 tetrahydrocannabidinolic acid (THCA) and Δ9 -tetrahydrocannabidinol.

13. A composition comprising:
- Lactobacillus paracasei subsp Paracasei F19 overexpressing four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
- caproic acids, or the salts and esters thereof,
- an ingestible vehicle edible suitable for the oral administration of the composition,
for the use as a medicament.

14. A composition comprising:
- Lactobacillus paracasei subsp Paracasei F19 overexpressing four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
- caproic acid, or salts and esters thereof,
- an ingestible vehicle suitable for oral administration of the composition,
for the use in the treatment of inflammatory bowel disorders.

15. A composition comprising:
- Lactobacillus paracasei subsp Paracasei F19 overexpressing four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
- caproic acid or salts and esters thereof,
- an ingestible vehicle suitable for oral administration of the composition,
for the use in the treatment of neurodegeneration diseases.

16. A composition comprising:
- Lactobacillus paracasei subsp Paracasei F19 overexpressing four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
- caproic acid or salts and esters thereof,
- an ingestible vehicle suitable for oral administration of the composition,
for the use in the treatment of colonrectal cancer.

17. Use of the composition according to claims 13 to 16 **characterized by** the fact to involve the administration of a daily dose in the range 12-24 x 10¹² - 12-24 x 10¹¹ cfu of *Lactobacillus Paracasei* subsp paracasei F19 overexpressing four *Cannabis sativa* genes selected in the group of:
(i) coding sequence for olivetol synthase (OLS) SEQ ID NO: 1,
(ii) coding sequence for olivetolic acid cyclase (OAC) SEQ ID NO: 2,
(iii) coding sequence for aromatic prenyltransferase (CsPT1) SEQ ID NO: 3,
(iv) coding sequence for cannabidiolic acid synthase (CAS) SEQ ID NO: 4, and
(v) coding sequence for tetrahydrocannabinolic acid synthase (THCAS) SEQ ID NO: 5.
and caproic acid in an amount between 0.25 and 0.35 µg in an ingestible vehicle.
